(19) Europäisches Patentamt
European Patent Office
Office européen des brevets

(11) **EP 2 574 280 A2**

(12) **EUROPEAN PATENT APPLICATION**

(43) Date of publication:
**03.04.2013 Bulletin 2013/14**

(51) Int Cl.:
**A61B 6/00** *(2006.01)*

(21) Application number: **12182417.1**

(22) Date of filing: **30.08.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (71) Applicant: **Fujifilm Corporation**<br>**Minato-ku**<br>**Tokyo (JP)** |
| (30) Priority: **28.09.2011 JP 2011212940** | (72) Inventor: **Tsuji, Tetsuya**<br>**Kanagawa (JP)** |
| | (74) Representative: **Klunker . Schmitt-Nilson . Hirsch**<br>**Patentanwälte**<br>**Destouchesstrasse 68**<br>**80796 München (DE)** |

(54) **Device, method, and program for assisting in initial setting of imaging condition, and radiation imaging apparatus and radiation imaging system**

(57)     Sample data (53) is stored in a console (24) of an electronic cassette (image detection panel) (21, 107). The sample data corresponds to a model of the electronic cassette and includes sample images captured using different doses before a shipment of the electronic cassette. In initial setting processing of an imaging condition including the dose, the sample data is read out and a dose selection screen (81) in which the sample images are arranged is displayed on a display (48). In the dose selection screen, the sample images are arranged in a tiled manner in an ascending or descending order of their doses (densities). A user compares the sample images to select the sample image with the density suitable for diagnosis. The dose corresponding to the selected sample image is set as an initial value.

FIG. 11

Printed by Jouve, 75001 PARIS (FR)

EP 2 574 280 A2

**Description**

BACKGROUND OF THE INVENTION

1. Field of the invention

The present invention relates to a device, method, and program for assisting in initial setting of an imaging condition in radiation imaging, and a radiation imaging apparatus and a radiation imaging system each having a function to assist in initial setting of an imaging condition.

2. Description Related to the Prior Art

In the medical field, imaging apparatuses using radiation, for example, X-rays are known. The X-ray imaging apparatus includes an image detection panel and a console. The image detection panel receives the X-rays, applied from an X-ray source and passed through a subject, to detect an X-ray image. The image detection panel outputs the X-ray image as digital data. The console sets imaging conditions including a dose. Examples of the image detection panel include an IP cassette using an imaging plate (IP) and an electronic cassette using a flat panel detector (FPD). Having advantages of using a computer for image processing and data instead of films, the image detection panels have become mainstream devices for X-ray imaging, replacing the conventional X-ray films.

The imaging conditions for the X-ray imaging, for example, a tube current and exposure time for determining a dose, and the tube voltage for determining X-ray quality (energy spectrum of the X-rays) are roughly determined by a body part to be imagined. However, each imaging condition for obtaining image quality suitable for diagnosis varies depending on a purpose of an examination, a physique of a subject, an interpretation skill of a doctor, or the like even if images of the same body part are captured. Therefore, the imaging condition roughly determined by the body part is used as an initial value. Then, in each imaging, the imaging condition is determined by slightly adjusting the initial value. The imaging condition is determined so as to reduce an exposure dose as much as possible without causing difficulty in diagnosis.

U. S. Patent No. 7, 949, 098 discloses a technique to assist in determining the imaging condition which maintains the image quality suitable for the diagnosis with a reduced exposure dose. The technique takes advantage of the X-ray image being the digital data. In the U. S. Patent No. 7,949,098, a past image is used as a basis image. The basis image is subjected to image processing. Thereby, two or more simulation images with different imaging conditions are displayed on a display.

The simulation images are evaluated by a specialist for interpretation, for example, a doctor. The simulation image with the image quality suitable for the diagnosis and the reduced exposure dose is selected. The imaging conditions of the selected image are determined to be used in the imaging. The method for determining the imaging conditions using the simulations with the image processing is very useful because it reduces the exposure dose when compared with a method for determining the imaging conditions through evaluation of the images of the subject captured with different imaging conditions. However, the method disclosed in the U. S. Patent No. 7, 949, 098 is not effective when an image detection panel of a new model is introduced instead of the model currently used, for example, typically, when a medical institution using the IP cassette introduces an electronic cassette.

The method disclosed in the U. S. Patent No. 7,949,098 uses the past image and the imaging condition of the past image as the basis of the simulation. However, when an image detection panel of a new model is introduced, a past image captured with the new model does not exist. Because the sensitivity of the image detection panel varies depending on the model, the density of the X-ray image varies depending on the model even if the same dose is given. It is difficult to simulate the imaging condition for the new image detection panel through image processing of the past image captured with the existing image detection panel. As a result, effective use of the new image detection panel is hampered, for example, the imaging condition corresponding to the existing image detection panel may be applied to the new image detection panel with high sensitivity even though the new image detection panel can be used in a different imaging condition with a reduced dose.

To solve the problems, preliminary imaging (test imaging) may be performed using the new image detection panel. The image obtained by the preliminary imaging is used as the basis image in the image simulation disclosed in the U. S. Patent No. 7, 949, 098. However, because the preliminary imaging for determining the imaging condition exposes the patient to unnecessary radiation, the preliminary imaging cannot be employed in view of dose reduction. The U. S. Patent No. 7, 949, 098 does not disclose or even suggest these problems and their solutions.

SUMMARY OF THE INVENTION

An object of the present invention is to provide a device, a method, and a program for assisting in initial setting of an imaging condition and a radiation imaging apparatus and a radiation imaging system capable of imaging with an imaging condition with a reduced dose in accordance with performance of an image detection panel without preliminary imaging.

A device for assisting in initial setting of an imaging condition according to the present invention includes a data storage means and a display control means. The data storage means stores sample data. The sample data corresponds to a model of the image detection panel. The sample data includes two or more sample images captured with different doses before a shipment of the image detection panel. In the initial setting, the display control means displays, on a display, the samples images read out from the data storage means so as to set an initial value of the dose.

It is preferable that a set of the sample images is prepared for each combination of a body part and a tube voltage, and the sample data includes two or more sets of the sample images. The tube voltage is supplied to the radiation source and determines radiation quality.

It is preferable that the sample data is prepared on a destination-by-destination basis of the shipment. The sample data may be prepared for each physique type of the subject.

It is preferable that the display control means displays a sample image screen on the display. It is preferable that the sample image screen displays the sample images in a tiled manner.

It is preferable that the sample image screen displays information representing the dose used for each of the sample images in addition to the sample images.

It is preferable that the sample data includes an S value calculated from a gray-level histogram of each of the sample images.

It is preferable that the device for assisting in initial setting of an imaging condition further includes a conversion means for converting the S value into an mAs value, being a product of a tube current supplied to the radiation source and exposure time.

It is preferable that the device for assisting in initial setting of an imaging condition further includes an SID receiving means and a correction means. The SID receiving means receives input of an SID, being a distance between the image detection panel and the radiation source. The correction means corrects the mAs value based on the inputted SID and a standard SID used for imaging the sample image.

It is preferable that the device for assisting in initial setting of an imaging condition further includes a selection instruction receiving means. The selection instruction receiving means receives input of a selection instruction that selects one of the sample images.

It is preferable that the display control means displays, on the display, at least information representing the dose of the sample image selected through the selection instruction receiving means.

A method for assisting in initial setting of an imaging condition includes a reading step and a displaying step. In the reading step, two or more sample images are read out from a data storage means. The data storage means stores sample data. The sample data corresponds to a model of the image detection panel. The sample data includes the sample images captured with different doses before a shipment of the image detection panel. In the displaying step, in the initial setting, the read-out sample images are displayed on a display to set an initial value of the dose.

A program for assisting in initial setting of an imaging condition includes a reading step and a displaying step. In the reading step, two or more sample images are read out from a data storage means. The data storage means stores sample data. The sample data corresponds to a model of the image detection panel. The sample data includes the sample images captured with different doses before a shipment of the image detection panel. In the displaying step, in the initial setting, the read-out sample images are displayed on a display to set an initial value of the dose.

A radiation imaging apparatus includes a console for setting an imaging condition including a dose of the radiation applied from the radiation source. The console includes a data storage means and a display control means. The data storage means stores sample data. The sample data corresponds to a model of the image detection panel. The sample data includes two or more sample images captured with different doses before a shipment of the image detection panel. The display control means displays on a display the sample images read out from the data storage means so as to set an initial value of the dose.

A radiation imaging system includes a console for setting an imaging condition including a dose of the radiation applied from the radiation source. The console includes a data storage means and a display control means. The data storage means stores sample data. The sample data corresponds to a model of the image detection panel. The sample data includes two or more sample images captured with different doses before a shipment of the image detection panel. The display control means displays on a display the sample images read out from the data storage means so as to set an initial value of the dose.

According to the present invention, sample data is stored in the data storage means before the shipment of the image detection panel. The sample data includes two or more sample images captured using the respective different doses and corresponds to the model of the image detection panel. To carry out the initial setting of the imaging condition, the sample images are displayed on the display after the image detection panel is introduced to the medical institution. A sample image suitable for diagnosis is selected from the sample images displayed. Without the preliminary imaging, the imaging is performed with the imaging condition with the reduced dose in accordance with the performance of the image detection panel.

BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:

Fig. 1 is a schematic view of an X-ray imaging system;
Fig. 2 is an explanatory view of a sequence of emission control;
Fig. 3 is a schematic view of an FPD;
Fig. 4 is a schematic view of a console;
Fig. 5 is an explanatory view of a processing block for carrying out initial setting processing and data used in the initial setting processing;
Fig. 6 is an explanatory view of an examination order screen;
Fig. 7 is an explanatory view of an initial value table;
Fig. 8 is a system diagram of sample data;
Fig. 9 is a graph describing sensitivity properties of electronic cassettes;
Fig. 10 is an explanatory view of an initial setting screen;
Fig. 11 is an explanatory view of a dose selection screen;
Fig. 12 is an explanatory view of an S value;
Fig. 13 is an explanatory view of a conversion table that converts an S value into an mAs value;
Fig. 14 is a schematic view of the initial setting processing;
Fig. 15 is a flowchart showing a workflow from production of the sample data to initial setting;
Fig. 16 is a flowchart showing steps of the initial setting;
Fig. 17 is a system view of another sample data different from that in Fig. 8;
Fig. 18 is an explanatory view of a dose selection screen of a second embodiment;
Fig. 19 is an explanatory view of a selection status screen;
Fig. 20 is an explanatory view showing processing to compare an average dose of past images with a dose after the initial setting;
Fig. 21 is an explanatory view of an X-ray imaging system of a fourth embodiment;
Fig. 22 is an explanatory view of another X-ray imaging system of the fourth embodiment, different from that in Fig. 21; and
Fig. 23 is an explanatory view of an example using a shared server.

DESCRIPTION OF THE PREFERRED EMBODIMENTS

(First embodiment)

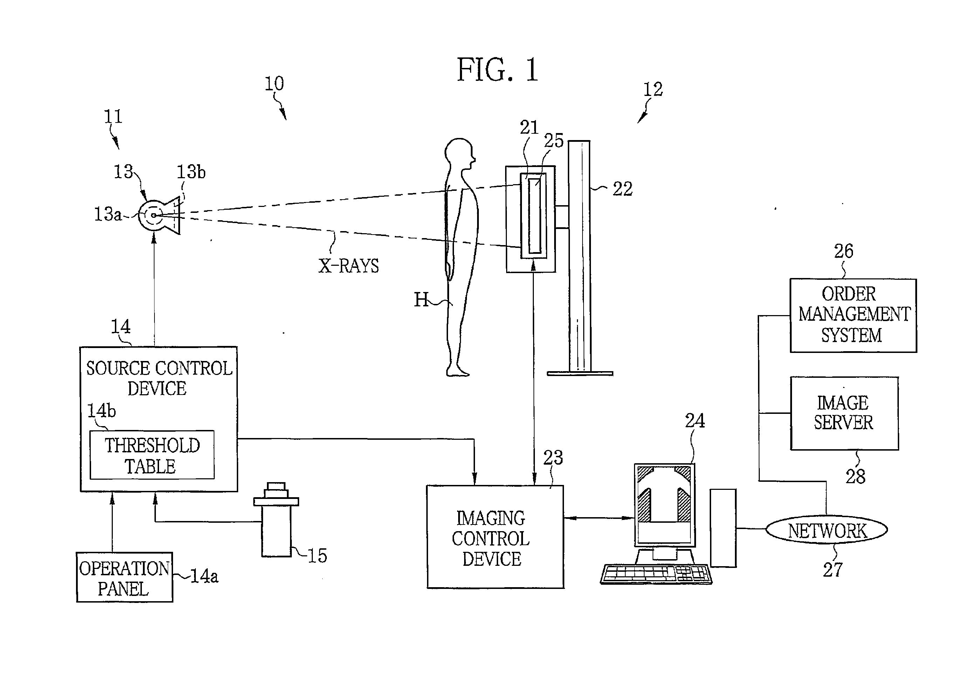

In Fig. 1, a radiation system, for example, an X-ray imaging system 10 is composed of an X-ray generating device 11 and an X-ray imaging apparatus 12. The X-ray generating device 11 is composed of an X-ray source 13, a source control device 14 for controlling the X-ray source 13, and a radiation switch 15. The X-ray source 13 has an X-ray tube 13a for emitting X-rays and a collimator 13b for limiting an X-ray field of the X-rays emitted from the X-ray tube 13a.

The X-ray tube 13a has a cathode and an anode (target). The cathode is composed of a filament that releases thermoelectrons. The target emits the X-rays when struck by the thermoelectrons released from the filament. The collimator 13b has a plurality of lead plates arranged in a lattice-like pattern to shield the X-rays. An opening for passing the X-rays is formed at the center of the lattice-like lead plates. The size of the opening is varied by moving the lead plates. Thus, the X-ray field is limited.

The source control device 14 is composed of a high voltage generator and a controller. The high voltage generator supplies high voltage to the X-ray source 13. The controller controls a tube voltage (unit: kV), a tube current (unit: mA), and X-ray radiation time (unit: second). The tube voltage determines X-ray quality (energy spectrum) of the X-rays applied from the X-ray source 13. The tube current (mA) determines a dose per unit time. The high voltage generator boosts an input voltage using a transformer to generate high tube voltage and supplies power to the X-ray source 13 through a high voltage cable. Through an operation panel 14a of the source control device 14, an operator, for example, a radiologist or a doctor manually sets imaging conditions such as the tube voltage and an mAs value, being a product of the tube current supplied to X-ray source 13 and the radiation time. The mAs value determines an exposure dose. The imaging conditions are stored in a threshold table 14b.

The radiation switch 15 is connected to the source control device 14 through a signal cable (not shown). The radiation switch 15 is a two-step push switch. When pressed one step, the radiation switch 15 generates a warm-up start signal for starting warm-up of the X-ray source 13. When pressed two steps down, the radiation switch 15 generates a radiation start signal for allowing the X-ray source 13 to start the X-ray emission. These signals are inputted to the source control device 14 through the signal cable.

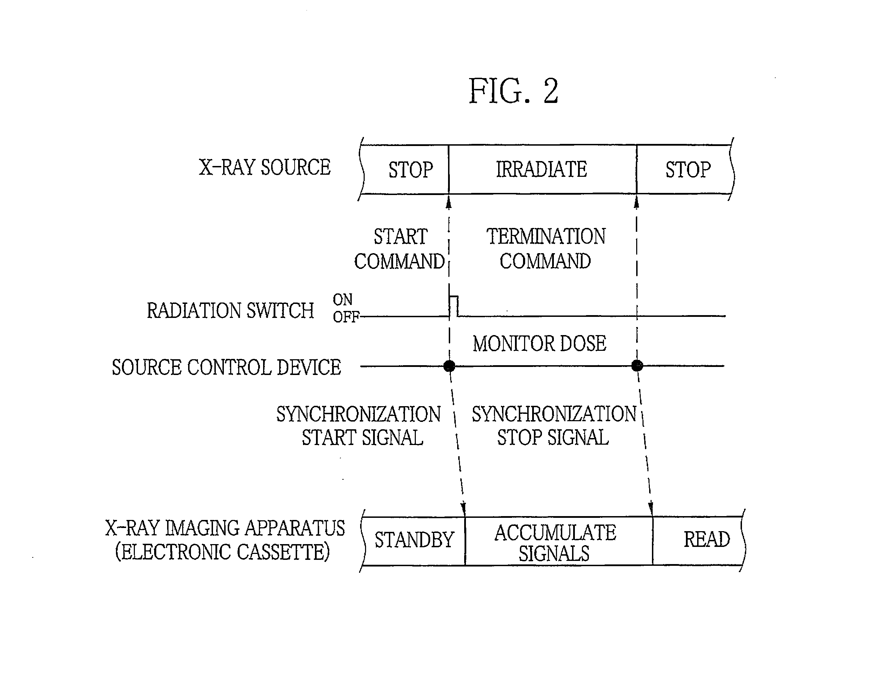

As shown in Fig. 2, the source control device 14 controls the operation of the X-ray source 13 based on the control signal from the radiation switch 15. Upon receipt of the radiation start signal (ON signal) from the radiation switch 15, the source control device 14 sends a start command to the X-ray source 13 and starts supplying the power to the X-ray source 13. Thereby, the X-ray source 13 starts application of the X-rays.

In Fig. 1, the threshold table 14b is a table memory for setting the imaging conditions. Two or more sets of initial values of commonly-used standard combinations of the tube voltage and the mAs value are preset in the threshold table 14b. To use a value other than the preset initial values, the value is inputted using a keyboard or a cursor key provided on the operation panel 14a. Thus, the imaging conditions are set.

In Fig. 2, after the start of the application of the X-rays, the source control device 14 refers to the threshold table 14b and monitors whether the actual exposure dose from the X-ray source 13 reaches a threshold value (mAs value) specified by the imaging conditions. Upon judging that the exposure dose has reached the threshold value, the source control device 14 sends a termination command to the X-ray source 13 to stop the application of the X-rays.

The source control device 14 is connected wirelessly or through a cable to the X-ray imaging apparatus 12 in a communicable manner. The source control device 14 sends to the X-ray imaging apparatus 12 a synchronization signal to synchronize irradiation timing of the X-ray source 13 and the operation timing of the X-ray imaging apparatus 12. The source control device 14 sends the start command to the X-ray source 13 and a synchronization start signal to the X-ray imaging apparatus 12, simultaneously. The source control device 14 sends the termination command to the X-ray source 13 and a synchronization stop signal to the X-ray imaging apparatus 12, simultaneously. Upon receipt of the synchronization start signal, the X-ray imaging apparatus 12 changes over from a standby state to an accumulation operation to accumulate signal charge corresponding to an amount of the incident X-rays. Upon receipt of the synchronization stop signal, the X-ray imaging apparatus 12 starts a reading operation to read the signal charge accumulated.

In Fig. 1, the X-ray imaging apparatus 12 is composed of an electronic cassette 21, an X-ray stand 22, an imaging control device 23, and a console 24. The electronic cassette 21 is composed of an FPD 25 and a portable housing for accommodating the FPD 25. The electronic cassette 21, being a portable radiation image detection device, receives the X-rays applied from the X-ray source 13 and then passed through an examinee (subject or patient) H to detect an X-ray image of the examinee H. The housing of the electronic cassette 21 has a flat shape with a substantially rectangular plane. The size of the plane is substantially the same as that of a film cassette or an IP cassette.

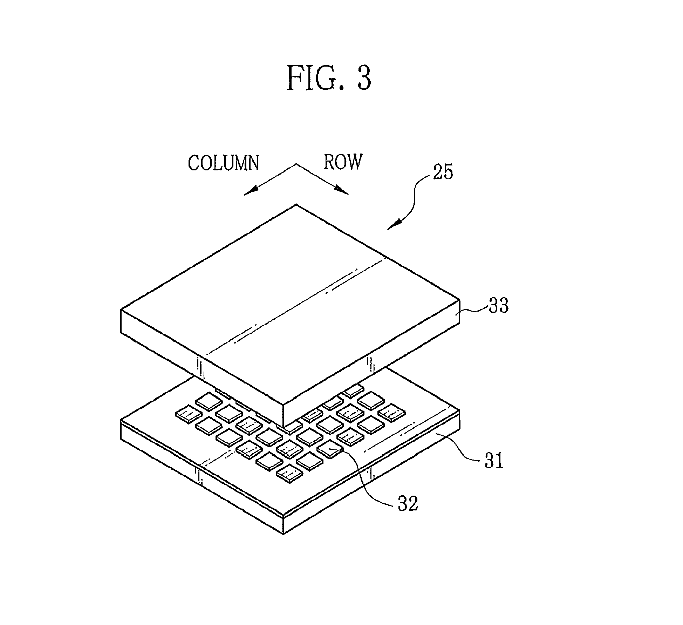

In Fig. 3, the FPD 25 has a TFT active matrix substrate 31 and an imaging area over the substrate 31. A plurality of pixels 32 are arranged in the imaging area. Each pixel 32 accumulates signal charge corresponding to the amount of the X-rays incident thereon. The FPD 25 is an indirect conversion type that is provided with a scintillator 33 over the substrate 31. The scintillator 33 converts the incident X-rays into visible light. Then, the pixels 32 photoelectrically convert the visible light into the signal charge. Each pixel 32 is composed of a photodiode and a TFT. The photodiode is a photoelectric conversion element. The TFT is a switching element used to read out the signal charge converted by the photoelectric conversion element. The pixels 32 are arranged at a predetermined pitch in a two-dimensional matrix. To the substrate 31, a gate driver and a controller (not shown) are connected. The gate driver drives the TFTs to control the reading out of the signal charge. The controller includes a signal processing circuit that converts the read-out signal charge into digital data and outputs the digital data.

The scintillator 33 is placed to face the whole imaging area in which the pixels 32 are arranged. The scintillator 33 is phosphor, for example, CsI (cesium iodide) or GOS (gadolinium oxysulfide). Note that a direct conversion type FPD may be used instead of the indirect conversion type. The direct conversion type FPD uses a conversion layer (for example, amorphous selenium) that directly converts the X-rays into charge.

The X-ray stand 22 has a slot to which the electronic cassette 21 is detachably attached. The X-ray stand 22 holds the electronic cassette 21 such that an X-ray incident surface of the electronic cassette 21 faces the X-ray source 13. Because the size of the housing of the electronic cassette 21 is substantially the same as that of the film cassette or the IP cassette, the electronic cassette 21 can be attached to an X-ray stand designed to use the film cassette or the IP cassette. Note that the X-ray stand 22 that supports the examinee H in a standing position is used by way of example. Alternatively, an X-ray table that supports the examinee H in a lying position may be used.

The imaging control device 23 is connected wirelessly or through a cable to the electronic cassette 21 in a communicable manner to control the electronic cassette 21. To be more specific, the imaging control device 23 sends the imaging conditions to the electronic cassette 21. Thereby, conditions of signal processing in the FPD 25 is set. The imaging control device 23 receives from the X-ray generating device 11 the synchronization signals (the synchronization start signal and the synchronization stop signal) for synchronizing the irradiation timing of the X-ray source 13 and the operation of an FPD 36, and sends the synchronization signals to the electronic cassette 21. Thereby, the imaging control device 23 controls the synchronization of the X-ray source 13 and the FPD 36. The imaging control device 23 receives the image data outputted from the electronic cassette 21 and sends the image data to the console 24.

The console 24 receives input of an examination order and displays the examination order on a display. The examination order is inputted manually by an operator or from an order management system 26, for example, an HIS (Hospital Information System) or RIS (Radiology Information System) through a network 27 such as a LAN. The order management system 26 manages patient information and examination information related to radiological examination.

The examination order is produced for each patient. Each examination order includes client information (a doctor ID, a name of a clinical department, or the like), patient information (a patient's name, a patient ID, or the like), and one or more X-ray imaging requests. Each X-ray imaging request includes technique information that specifies imaging details such as a body part (for example, head, chest, or stomach) to be imaged, an imaging direction, and an imaging posture (for example, the standing position or the lying position). Examples of the imaging directions include, front (anterior), lateral, oblique, P-A (postero-anterior, meaning "from the back toward the front", namely, the X-rays are applied from the back of the examinee H), and A-P (antero-posterior, meaning "from the front toward the back", namely, the X-rays are applied from the front of the examinee H). The operator checks the content of the X-ray imaging request on a display of the console 24 and inputs the imaging conditions corresponding to the X-ray imaging request through an operation screen of the console 24.

The console 24 sends the imaging conditions to the imaging control device 23. The console 24 performs various image processing steps, for example, gamma correction and frequency processing, to data of the X-ray image sent from the imaging control device 23. After being subjected to the image processing, the X-ray image is displayed on the display of the console 24. Additional information such as the imaging conditions and the patient information is added to the X-ray image, and then the X-ray image is converted into a data file of a medical image format such as DICOM (Digital Imaging and Communications in Medicine). The data file is stored in a hard disk or a memory in the console 24 or a data storage device such as an image server 28 connected to the console 24 through the network 27.

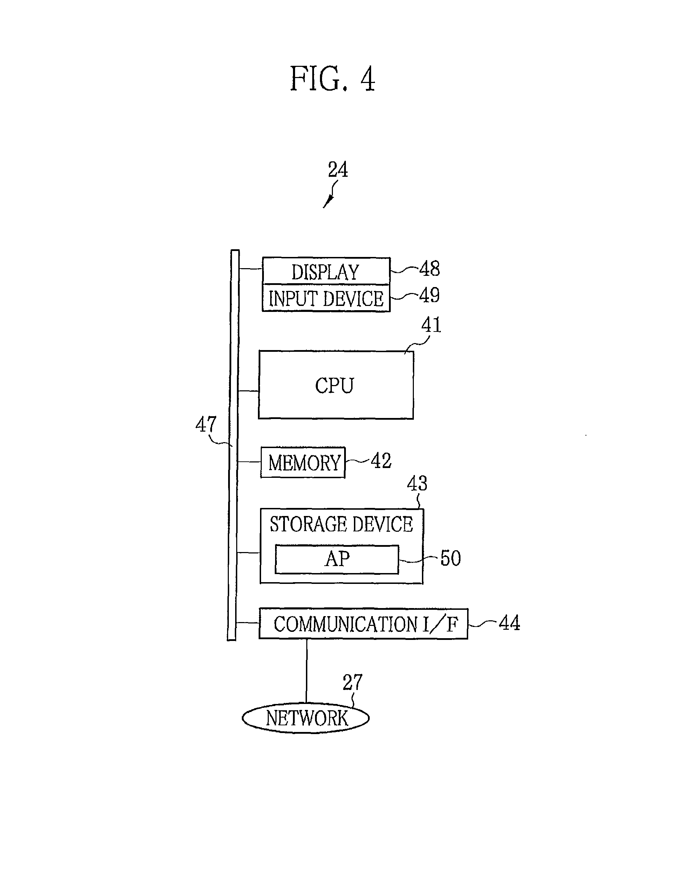

As shown in Fig. 4, the console 24 is a computer such as a personal computer or a work station installed with a control program such as an operating system or an application program (AP) 50 which allows the computer to function as the console 24.

The console 24 is provided with a CPU 41, a memory 42, a storage device 43, a communication I/F 44, a display 48, and an input device 49, which are connected to each other via a data bus 47. The input device 49 includes a keyboard and a mouse. A touch panel type input device integrated with the display 48 may be used.

The storage device 43 is, for example, a hard disk drive, being an internal storage device incorporated in a body of the console 24. The storage device 43 may be an external storage device connected to the body of the console 24 through a network or a communication cable. The storage device 43 stores, for example, the control program and the application program (AP) 50 such as software for the console 24.

The memory 42 is a working memory used by the CPU 41 to carry out the processing. The CPU 41 loads the control program stored in the storage device 43 to the memory 42 and carries out the processing according to the program. Thus, the CPU 41 controls each part of the computer. The communication I/F 44 has a network I/F that controls transmission between the computer and the network 27 such as the LAN. The console 24 communicates with the image server or the order management system 26 such as the RIS or the HIS through the network 27. The communication I/F 44 is also provided with an I/F to communicate with the electronic cassette 21 through the imaging control device 23.

The software for the console 24 is a program that allows the computer to perform functions, such as displaying the examination order or the X-ray image sent from the electronic cassette 21 on the display 48, performing image processing to the X-ray image, and setting the imaging condition(s) corresponding to the imaging request. The function to set the imaging condition includes a function to assist setting of an initial value of the imaging condition.

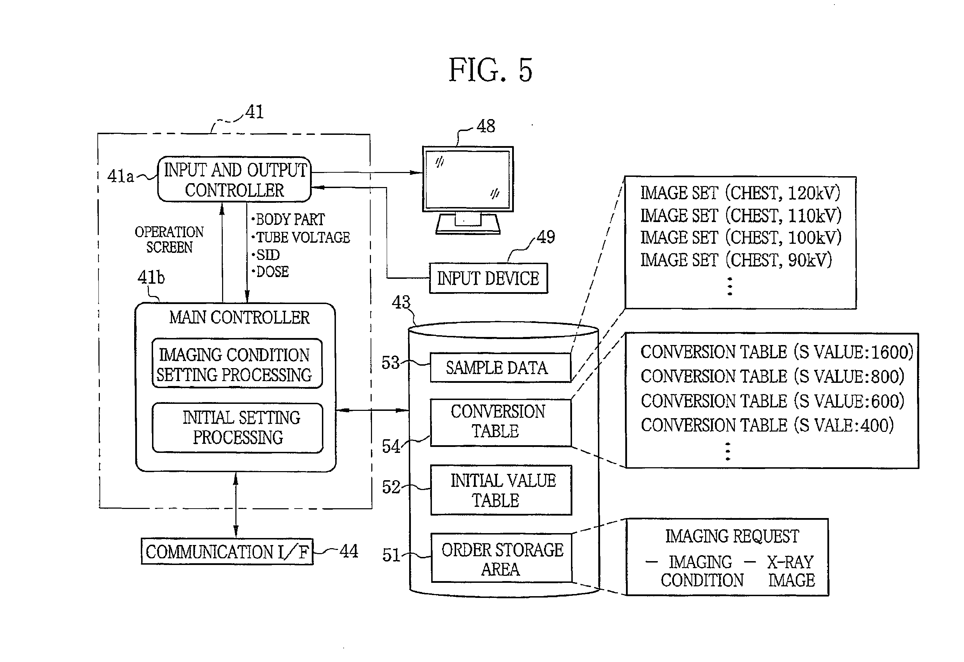

As shown in Fig. 5, when the software for the console 24 runs, the CPU 41 together with the memory 42 function as an input and output controller 41a and a main controller 41b. The input and output controller 41a controls information outputted to the display 48 and information inputted from the input device 49. To the display 48, the input and output controller 41a outputs the X-ray image captured with the electronic cassette 21 and the operation screen produced using GUI (graphical use interface). The input and output controller 41a receives an operation instruction inputted from the input device 49 with the use of the operation screen 48.

The main controller 41b communicates with the electronic cassette 21 through the communication I/F 44 and the imaging control device 23. Thereby, the main controller 41b performs cassette control processing for controlling the electronic cassette 21 and image processing to the X-ray image inputted from the electronic cassette 21. The main controller 41b also performs examination order receiving processing, imaging condition setting processing, and initial setting processing. In the examination order receiving processing, the main controller 41b receives the examination order inputted from the order management system 26, the operation screen, or the like. In the imaging condition setting processing, the main controller 41b sets the imaging condition for each imaging request contained in the examination order based on the operation instruction inputted from the operation screen. In the initial setting processing, the main controller 41b sets the initial value of the imaging condition.

The storage device 43 is provided with an order storage area 51 for storing the imaging request. In the imaging condition setting processing, when one or more imaging conditions for the imaging request are set, the imaging conditions are stored in the order storage area 51 in association with the imaging request. When the imaging corresponding to the imaging request is performed and the X-ray image is inputted to the storage device 43, the X-ray image is stored in association with the imaging request in the order storage area 51. Thus, the imaging request, the imaging condition(s), and the X-ray image are associated with each other. The X-ray image is converted into the DICOM format and then sent to the image server 28 through the communication I/F 44.

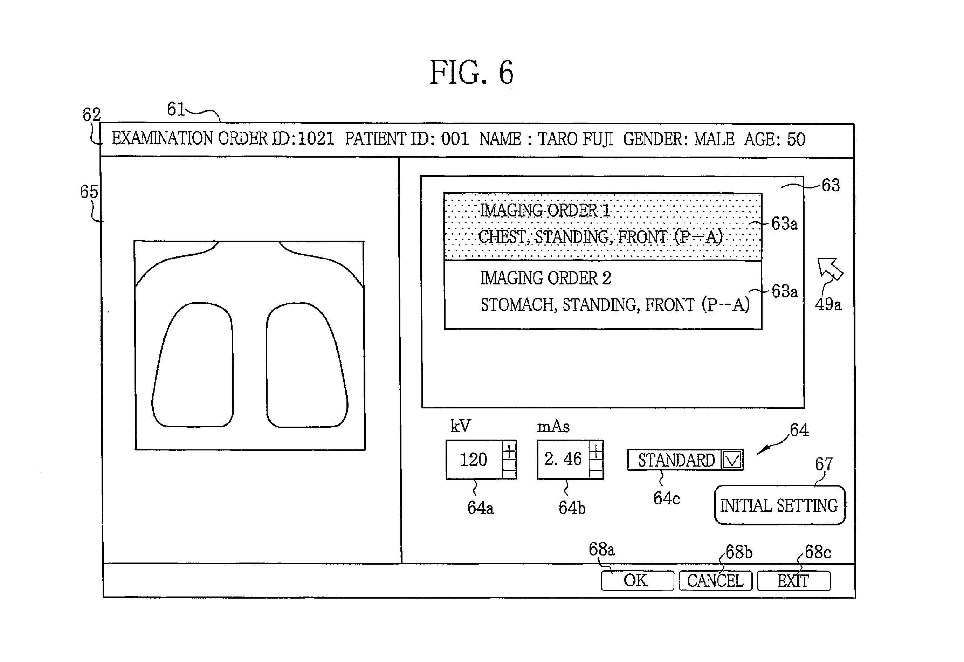

In the imaging condition setting processing, an examination order screen 61 (see Fig. 6), being the operation screen, is displayed on the display 48. The examination order screen 61 selectively displays one of the examination orders received.

The examination order screen 61 is provided with a patient information area 62, an imaging request area 63, and an image display area 65. The patient information area 62 displays the patient information including, for example, an examination order ID, a patient's name, a patient ID, gender, and age. The imaging request area 63 displays the imaging request(s) of the single examination order. In the image display area 65, the X-ray image captured is displayed. An imaging condition input area 64 is provided below the imaging request area 63. The imaging condition(s) corresponding to the selected imaging request is inputted to the imaging condition input area 64. An initial setting button 67 is used to invoke an initial setting screen for setting an initial setting of the imaging condition. An OK button 68a is used to confirm the inputted imaging condition(s). A cancel button 68b is used to cancel the inputted imaging condition(s). An exit button 68c is used to exit the examination order screen 61.

When there are two or more imaging requests in the imaging request area 63, all the imaging requests are displayed in a list form. In this example, two imaging requests are included in the single examination order. In the imaging request area 63, each display box 63a of the imaging request displays the technique information of the imaging request, for example, "chest, standing position, P-A".

Clicking one of the display boxes 63a with a pointer 49a of a mouse of the input device 49 selects the imaging request corresponding to the selected display box 63a. The selected imaging request (the selected display box 63a) is highlighted to be distinguished from unselected imaging request(s). In this example, the imaging request "1" is selected.

In the image display area 65, the latest one of the captured X-ray images is displayed. In this example, the X-ray image corresponding to the imaging request 1 is displayed. Alternatively, an X-ray image selected from the captured X-ray images may be displayed in the image display area 65.

The imaging condition input area 64 is provided with a tube voltage input box 64a, an mAs value input box 64b, and a physique information input box 64c. Clicking one of the boxes 64a to 64c with the pointer 49a of the mouse makes the selected box active and ready to accept input of information. A keyboard may be used to input a numerical value to the tube voltage input box 64a. Alternatively, two or more numerical values (for example, 120kV, 110kV, 100kV, and 90kV) may be listed in a pull-down menu. One of the numerical values is selected using the pointer 49a and inputted.

When the tube voltage is inputted into the tube voltage input box 64a, an initial value of the mAs value is automatically input to the mAs value input box 64b. The initial value of the mAs value is previously set for each combination of the body part and the tube voltage. In this example, according to the imaging request 1 selected, the body part to be imaged is "chest", and the tube voltage inputted is "120 kV". Thereby, the initial value "2.46" of the mAs value corresponding to the combination of the chest and 120kV is inputted automatically. At an edge of each of the boxes 64a and 64b, adjustment buttons ("+" and "-") are provided to increase or decrease the input value.

Physique information (body size information) of the examinee H is inputted to the physique information input box 64c. The pull-down menu is provided with options, for example, "standard", "obese", "thin", and "child". Attenuation of the X-rays varies based on the physique of the examinee H. Accordingly, the amount of the X-rays incident on the electronic cassette 21 varies depending on the physique of the examinee H even if the X-rays of the same exposure dose are applied from the X-ray source 13. For this reason, the mAs value that determines the exposure dose is adjusted based on the inputted physique information. To be more specific, for example, when the "obese" is selected, the mAs value is adjusted to be twice or three times the mAs value of the "standard". When the "thin" is selected, the mAs value is adjusted to be half the mAs value of the "standard". When the "child" is selected, the mAs value is adjusted to be one third the mAs value of the "standard".

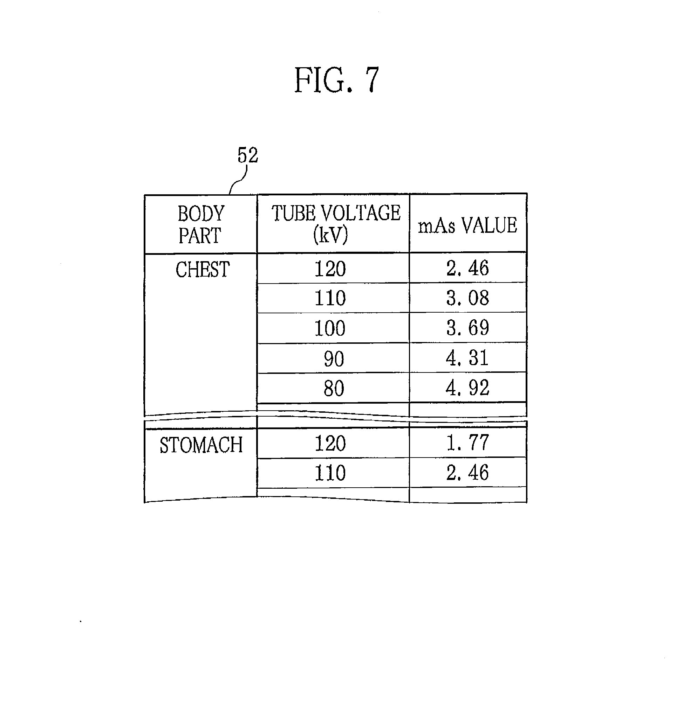

In Fig. 5, the storage device 43 stores an initial value table 52, sample data 53, and a conversion table 54. The conversion table 54 is used for converting an S value into an mAS value. As shown in Fig. 7, in the initial value table 52, the initial values of the mAs values are stored in association with the respective combinations of the body part and the tube voltage.

The mAs value determines the exposure dose for the X-ray imaging. A change in the mAs value mainly appears as a change in the density of the X-ray image. A small exposure dose reduces the dose absorbed by the examinee H. However, the small exposure dose also reduces the density of the X-ray image. As a result, graininess in the X-ray image increases. Generally, it is difficult for a doctor with a low level of interpreting skill to find a lesion in the X-ray image with increased graininess. On the other hand, although high exposure dose increases the dose absorbed by the examinee H, it also increases the density of the X-ray image. As a result, the graininess in the X-ray image decreases. Generally, it is easy to find a lesion in the X-ray image with the high density and less graininess. The density required for the X-ray image used for diagnosis varies depending on circumstances such as proficiency and preference of a doctor and a policy of a medical institution. The setting of the initial value of the mAs value in the initial value table 52 is changeable in accordance with the circumstances.

An appropriate exposure dose varies with the body part to be imaged. In addition, the appropriate exposure dose for the body part varies with the tube voltage. For this reason, in the initial value table 52, the initial value of the mAs value is stored in association with the combination of the body part and the tube voltage. The setting of the initial value in the initial value table 52 is changed in the initial setting processing.

The sample data 53 and the conversion table 54 are used in the initial setting processing. The sample data 53 is composed of two or more sample images captured with different exposure doses using the electronic cassette 21 before a shipment of the electronic cassette 21. A manufacturer of the electronic cassette 21 provides the sample data 53 to be used for determining the initial value of the exposure dose in the initial setting. The sample image shows the correspondence between the mAs value and the density of the X-ray image in using the electronic cassette 21. The user selects the sample image suitable for the diagnosis out of the sample images to set the initial value of the mAs value, which will be described later. A subject of the sample image may be a human body or a phantom having X-ray absorption properties similar to those of the human body.

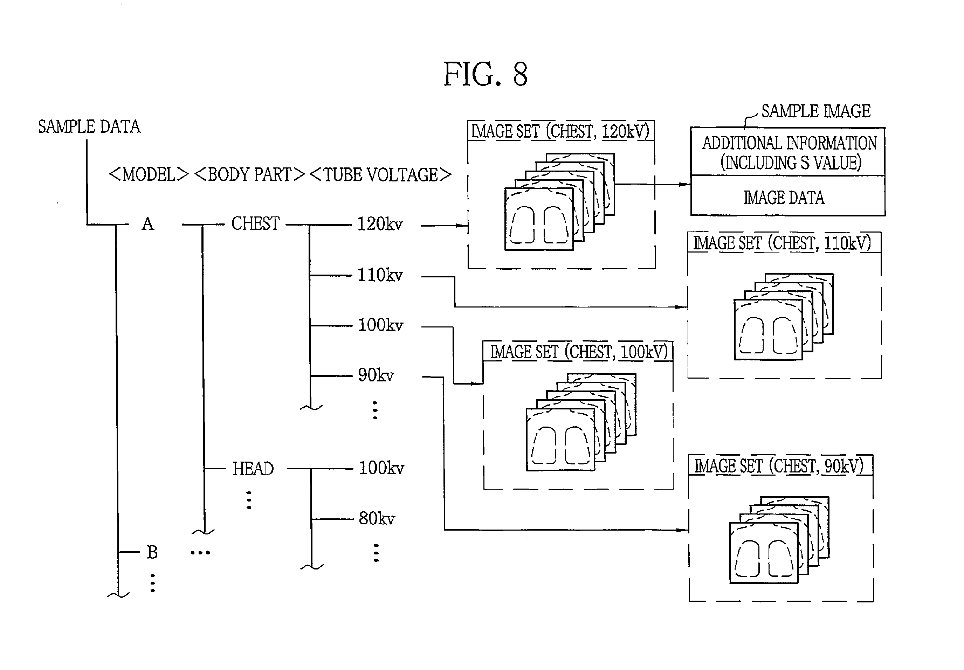

As shown in Fig. 8, the sample data 53 is prepared on a model-by-model basis of the electronic cassette 21. For example, each of a model A and a model B has different sample data 53. An initial value of the mAs value is set for each combination of the body part and the tube voltage. Namely, an image set composed of two or more sample images captured with different exposure doses is prepared for each combination. Each sample image in the image set is composed of image data, being a group of pixel values of pixels, and additional information. The additional information includes an S value of the corresponding sample image.

The S value is originally used as an index of reading sensitivity of the IP cassette. However, because there is a correlation between the S value and the dose (density), the S value is used as an index (hereinafter referred to as the dose index) to represent the dose, which will be described later. The mAs value represents the exposure dose from the X-ray source 13. The S value, on the other hand, is the dose index representing the dose (hereinafter may referred to as the detected dose) detected by the electronic cassette 21. The S value is obtained from a gray-level histogram of each of the sample images. For example, the manufacturer of the electronic cassette 21 calculates the S value and stores the S value as the additional information in the corresponding sample image.

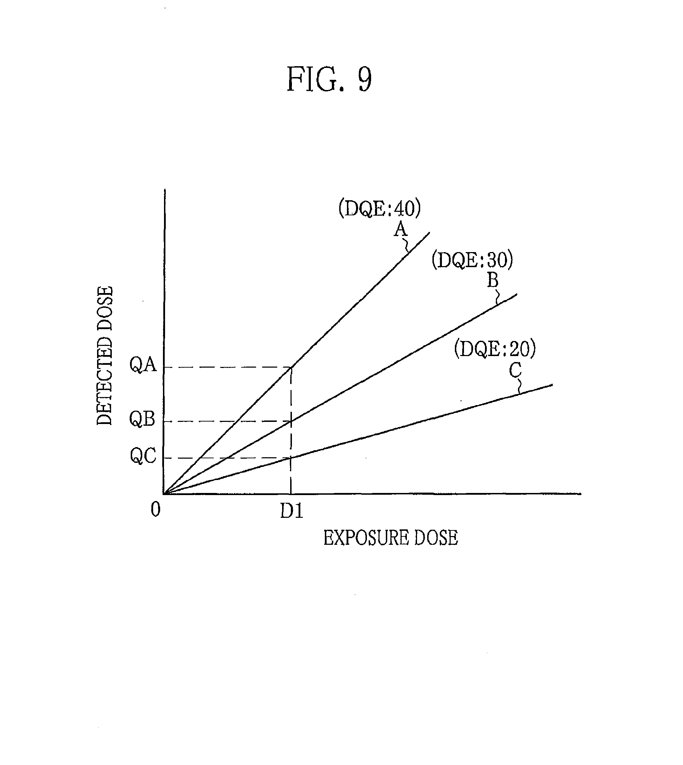

As shown in Fig. 9, DQE (Detective Quantum Efficiency) varies depending on a model (for example, A, B, or C) of the electronic cassette 21. The DQE is an index of the sensitivity of the electronic cassette 21. The detected dose increases (for example, QA> QB> QC) as the DQE increases even if the exposure dose (D1) is unchanged. In other words, the X-ray image is obtained with the lower exposure dose without changing the density of the X-ray image. The IP cassette and the electronic cassette have different DQEs. The DQE of the electronic cassette is generally higher than that of the IP cassette. The direct conversion type electronic cassette and the indirect conversion type electronic cassette have different DQEs. The DQEs vary depending on models of the indirect conversion type electronic cassettes, because sensitivity properties of the photodiodes of the FPDs 25 and types of the scintillators 33 vary depending on the models. The sample data 53 is prepared on a model-by-model basis so as to set the initial value of the mAs value in accordance with the sensitivity property of the electronic cassette 21.

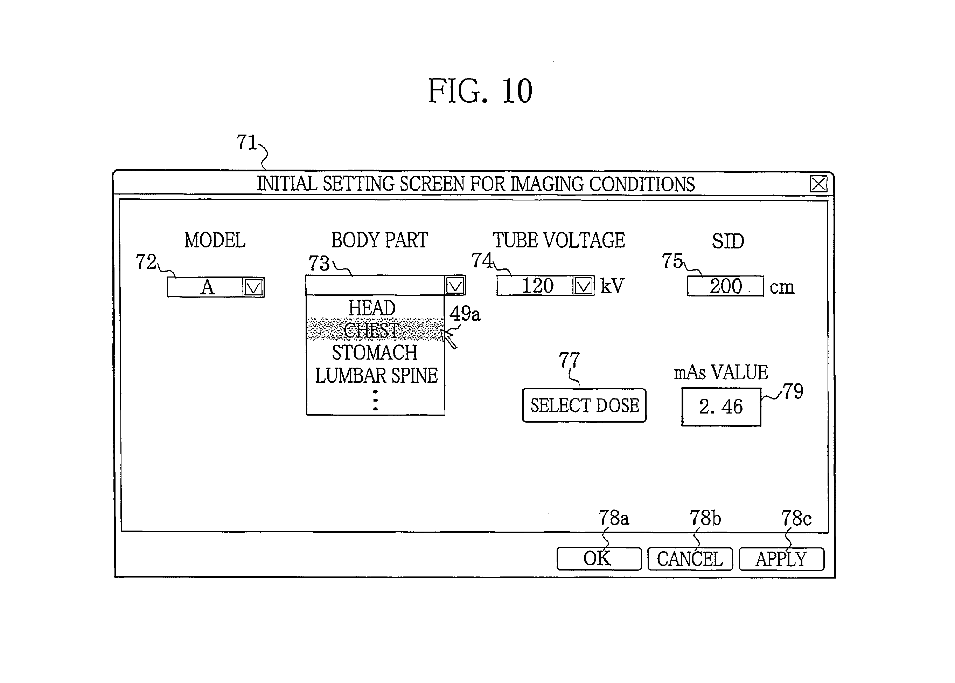

In the initial setting processing, as shown in Fig. 10, an initial setting screen 71, being the operation screen, of the imaging conditions is displayed on the display 48. Clicking the initial setting button 67 of the examination order screen 61 invokes the initial setting screen 71.

The initial setting screen 71 is provided with a model selection box 72, a body part selection box 73, a tube voltage selection box 74, and an SID input box 75. As shown by the body part selection box 73 by way of example, each of the selection boxes 72 to 74 is provided with a pull-down menu. One of the options of the pull-down menu is selected with the pointer 49a and inputted. The model of the electronic cassette 21 to be used is inputted into the model selection box 72. The body part to be imaged is inputted into the body part selection box 73. The tube voltage is inputted into the tube voltage selection box 74. The combination of the body part to be imaged and the tube voltage determines the initial value of the mAs value.

A distance (SID; Source Image Distance, unit: cm) between the X-ray source 13 and an imaging surface of the electronic cassette 21 (FPD 25) in the X-ray imaging system 10 in the medical institution is inputted to the SID input box 75 using the keyboard. The SID varies depending on an installation environment of the X-ray imaging system 10 in the medical institution. When there are two or more X-ray imaging systems 10 in the medical institution, the SID varies depending on the installation environment of each X-ray imaging system 10. The SID value inputted to the SID input box 75 is used as a parameter for distance correction of the mAs value, which will be described later.

An OK button 78a, a cancel button 78b, and an apply button 78c are operation buttons for the selection boxes 72 to 74 and the SID input box 75. Each of the OK button 78a and the apply button 78c is used to confirm the information inputted to the selection boxes 72 to 74 and the SID input box 75. Clicking the OK button 78a with the pointer 49a confirms the information inputted, and at the same time, the initial setting screen 71 is exited and the examination order screen 61 appears. Clicking the apply button 78c with the pointer 49a also confirms the inputted information. In this case, on the other hand, the initial setting screen 71 remains displayed. The information confirmed is sent to the main controller 41b through the input and output controller 41a. Clicking the cancel button 78b with the pointer 49a exits the initial setting screen 71 without confirming the inputted information.

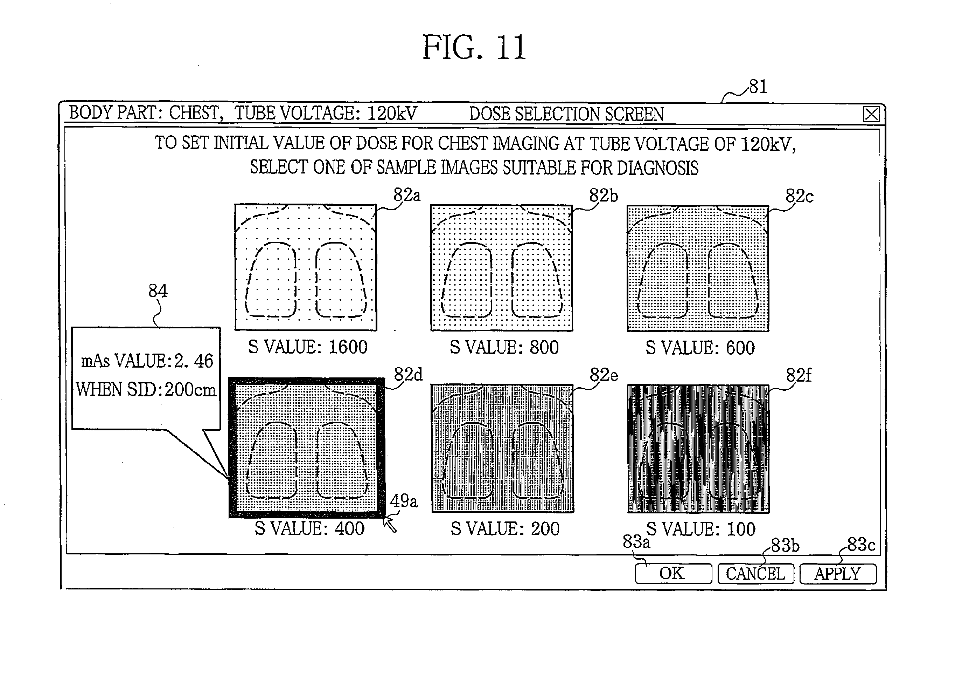

A dose selection button 77 is used to display a dose selection screen 81 (see Fig. 11). The dose selection screen 81 is used to select the mAs value (dose) corresponding to the combination of the body part and the tube voltage selected. A display area 79 displays a dose selected through the dose selection screen 81 and set in the initial value table 52. In Fig. 10, the dose selected is displayed in the initial setting screen 71. The dose (the mAs value: 2.46), being the initial value corresponding to the imaging conditions inputted (model: A, body part: chest, tube voltage: 120kV, SID: 200 cm) is read out from the initial value table 52 and displayed in the display area 79. The display area 79 is blank when the initial value is not set, for example, before the first initial setting immediately after the electronic cassette 21 is installed in the medical institution.

As shown in Fig. 11, sample images 82a to 82f, captured with different exposure doses, are displayed in a tiled manner in the dose selection screen 81 so as to be compared. The sample images 82a to 82f displayed in the dose selection screen 81 are included in the image set (see Fig. 8) corresponding to the combination (the body part and the tube voltage) selected using the selection boxes 73 and 74 in the initial setting screen 71. In an upper portion of the dose selection screen 81, the combination selected (in this example, "chest" and "120kV") is displayed.

Out of the sample images 82a to 82f, the sample image 82a has the lowest dose (that is, the lowest density), and the dose (density) increases in the order of the sample images 82b, 82c, 82d, 82e, and 82f (the highest). In the initial setting, the doctor evaluates the sample images 82a to 82f, and then selects one of the sample images 82a to 82f with the density suitable for the diagnosis, based on the proficiency and preference of the doctor and the policy of the medical institution.

When the sample image (in this example, the sample image 82d) is selected using the pointer 49a in the dose selection screen 81, a frame-shaped cursor is displayed around the sample image 82d, for example. Thus, the selected sample image 82d is distinguished from the rest of the sample images. When one of the sample images is selected, the main controller 41b performs an mAs value calculation processing to calculate the dose (mAs value) corresponding to the sample image selected.

The mAs value calculation processing includes conversion processing and distance correction processing, which will be described later. In the conversion processing, the S value stored in the additional information of the sample image is converted into the mAs value. In the distance correction processing, the mAs value is subjected to the distance correction based on the SID inputted through the initial setting screen 71. A speech balloon-shaped area 84 is displayed close to the sample image 82d selected. The mAs value calculated in the mAs value calculation processing is displayed in the speech balloon-shaped area 84. Thereby, the mAs value corresponding to the selected sample image is displayed to the user in the medical institution. The user checks the exposure dose (the mAs value) necessary for capturing the X-ray image, having the same density as the selected sample image, using the electronic cassette 21.

Each of an OK button 83a and an apply button 83c is used to confirm the selection. Clicking the OK button 83a with the pointer 49a confirms the selection. At the same time, the dose selection screen 81 is exited and the initial setting screen 71 appears. Clicking the apply button 83c confirms the selection, but the dose selection screen 81 remains displayed. Clicking a cancel button 83b exits the dose selection screen 81 without confirming the selected information.

When the selection is confirmed using the OK button 83a or the apply button 83c, the dose (mAs value) corresponding to the confirmed sample image is stored as the initial value of the mAs value in the initial value table 52. The initial value of the mAs value corresponds to the selected imaging condition, that is, the combination of the body part and the tube voltage.

The S value, being the dose index, is displayed below each of the sample images 82a to 82f. As described above, each of the sample images 82a to 82f has the S value, being the additional information. The S value is read out and displayed in the dose selection screen 81. The user refers to the S values in addition to the sample images to select the appropriate density.

The S value is originally used as an index of the reading sensitivity of the IP cassette. To use the IP cassette in radiation imaging, as is well-known, image data is read out by scanning the IP cassette with visible light after the IP cassette is irradiated with radiation. Before the scanning (main scanning) to read out the image data, pre-scanning (test scanning) is performed to determine an amount of light used for the main scanning. A gray-level histogram of the image data obtained by the pre-scanning is used to determine the reading sensitivity in the main scanning. Because there is the correlation between the S value and the dose (density), which will be described in the following, the S value is used as the dose index in this example.

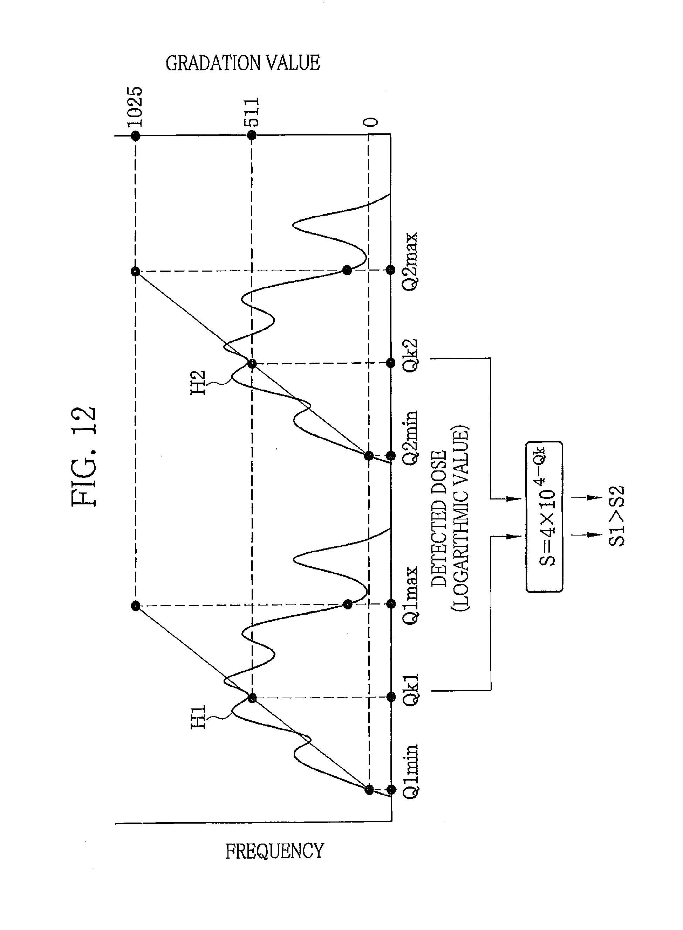

As shown in Fig. 12, the S value is obtained from a gray-level histogram of the image data. In Fig. 12, two gray-level histograms H1 and H2 are obtained from image data of respective two images of the same body part captured at the same tube voltage, but with different doses. Accordingly, the shapes of the two histograms are the same, but the average densities thereof are different. In the gray-level histogram, it is considered that a directly exposed area, in which the X-rays are incident directly on the image detection panel without passing through the subject (the examinee H), has the maximum density. An effective range (Qmin to Qmax) that is to be converted into digital gradation values is determined. Thereby, a dynamic range corresponding to the bit number of the gradation values is effectively used. Thus, an image with high contrast is obtained.

When each of the image data with the gray-level histogram H1 and the image data with the gray-level histogram H2 is converted into the gradation value of 10 bits, the Qmin corresponds to "0" and the Qmax corresponds to "1025". The S value is defined by an expression (1) S value $=4 \times 10^{4-Qk}$. The "Qk" corresponds to "511" that is a median of the gradation values. When the gray-level histograms H1 and H2 are compared with each other, each of the Qk values (Qk1 and Qk2) is proportional to the dose (density). The S values (S1 and S2) are reciprocals of the Qk values (Qk1 and Qk2), respectively. Accordingly, S1>S2 when Qkl<Qk2. Namely, the S value decreases as the dose (density) increases. The S value increases as the dose (density) decreases.

Namely, out of the sample images 82a to 82f in the dose selection screen 81 shown in Fig. 11, the sample image 82a with the lowest density has the highest S value (1600). The sample image 82f with the highest density has the lowest S value (100).

The mAs value set in the initial value table 52 determines the exposure dose from the X-ray source 13. The S value, on the other hand, corresponds to the dose detected by the electronic cassette 21. In other words, the S values takes account of an amount of the X-rays absorbed by the examinee H. For this reason, the conversion table 54 (see Fig. 5) stored in the storage device 43 is used to convert the S value into the mAs value.

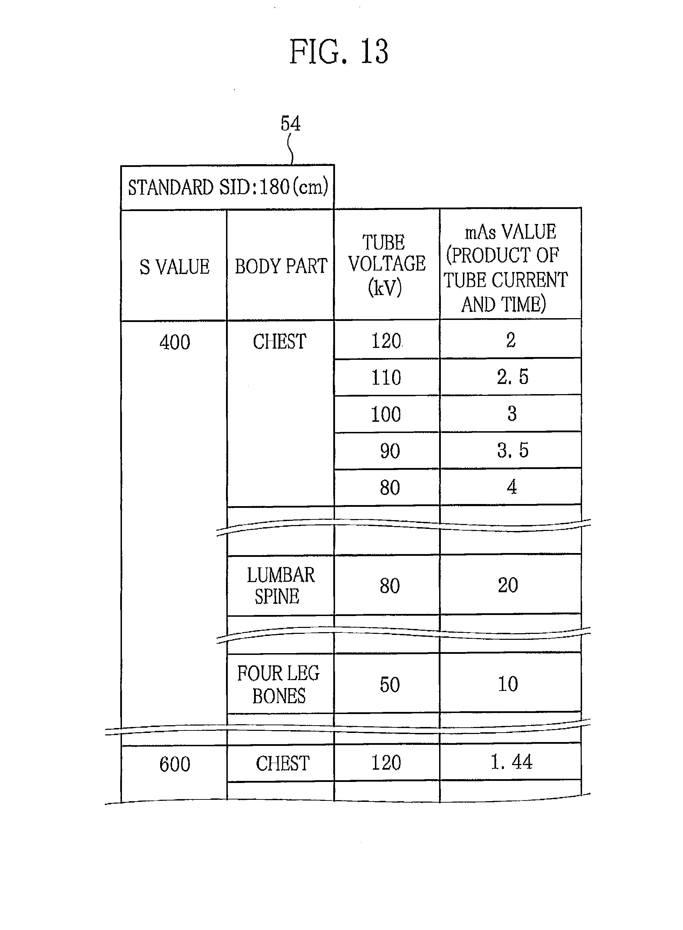

As shown in Fig. 13, in the conversion table 54, each of the S values (for example, 400, 600, and so forth) is provided and associated with two or more combinations of the body part and the tube voltage, and each combination is provided with the corresponding mAs value. The manufacturer of the electronic cassette 21 produces the conversion table 54 along with the sample data.

To be more specific, the manufacturer stores an mAs value, obtained at the time of the image capture of the sample image, for each combination of the body part and the tube voltage. To capture the sample images, a subject similar to the examinee H of the standard physic is used. An image analyzing device obtains the gray-level histogram of each sample image to calculate the S value. The S value is stored in the additional information of the corresponding sample image. The conversion table 54 is produced using the S value and the mAs value. A single SID is used for the imaging of all the sample images, for example, and stored in the conversion table 54 as a standard SID (in this example, 180 cm). The standard SID may vary depending on the combination of the body part and the tube voltage. In this case, the standard SID is stored for each combination.

In the initial setting processing, the mAs value calculation processing is performed using the conversion table 54. The mAs value calculation processing includes the conversion processing and the distance correction processing. In the conversion processing, the main controller 41b refers to the conversion table 54 to read out the appropriate mAs value based on the combination (the body part and the tube voltage) selected through the initial setting screen 71 and the S value read out from the additional information of the sample image selected through the dose selection screen 81.

The X-ray intensity from the X-ray source 13 attenuates in inverse proportion to a square of a distance. The mAs value determines the exposure dose from the X-ray source 13. Accordingly, when the SID used in producing the sample data and the SID used in the medical institution where the electronic cassette 21 is installed are different, it is necessary to perform the distance correction processing to correct the mAs value based on a ratio between the SIDs.

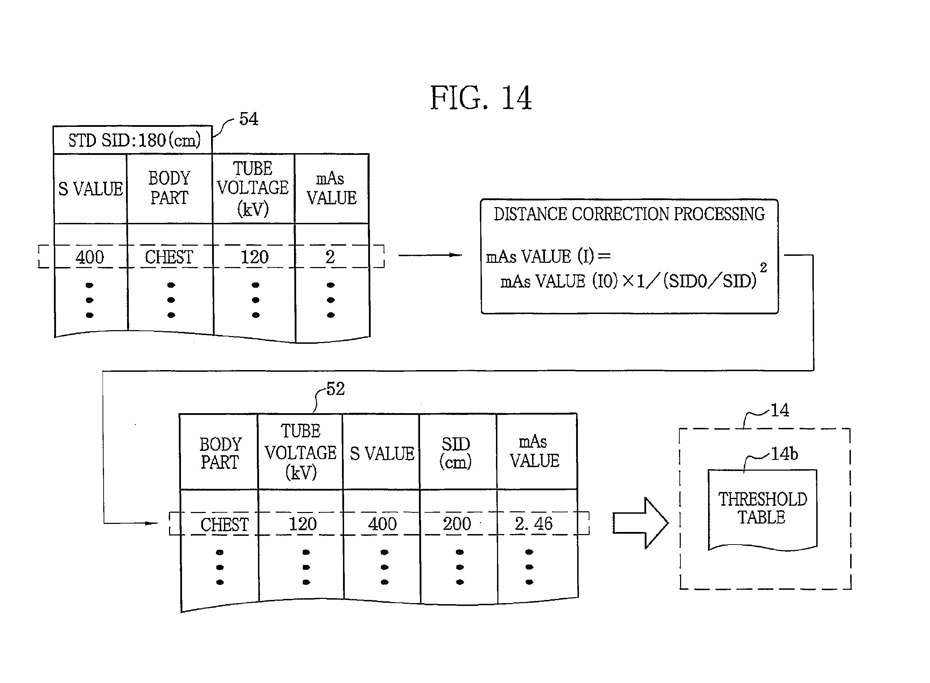

As shown in Fig. 14, using an expression (2), the main controller 41b performs the distance correction processing to the mAs value read out from the conversion table 54.

$$(2) \ \ \text{mAs value } (I) = \text{mAs value } (I0) \times 1/(SID0/SID)^2$$

The "mAs value (10)" denotes the mAs value read out from the conversion table 54 and not being subjected to the adjustment. The "SID0" denotes the standard SID. The "SID" denotes a value inputted through the initial setting screen 71. For example, the mAs value (I) is "2.46" when the SID0 is "180 cm", the SID is "200 cm", and the mAs value (10) is "2".

Thus, the S value is obtained from the mAs value through the mAs value calculation processing. As described above, the input and output controller 41a displays the calculated mAs value in the speech balloon-shaped area 84 when one of the sample images is selected through the dose selection screen 81. When the selection is confirmed, the main controller 41b stores the mAs value in the initial value table 52 after the distance correction processing. These steps are performed for each combination of the body part and the tube voltage. Thus, the initial values of the mAs values are set.

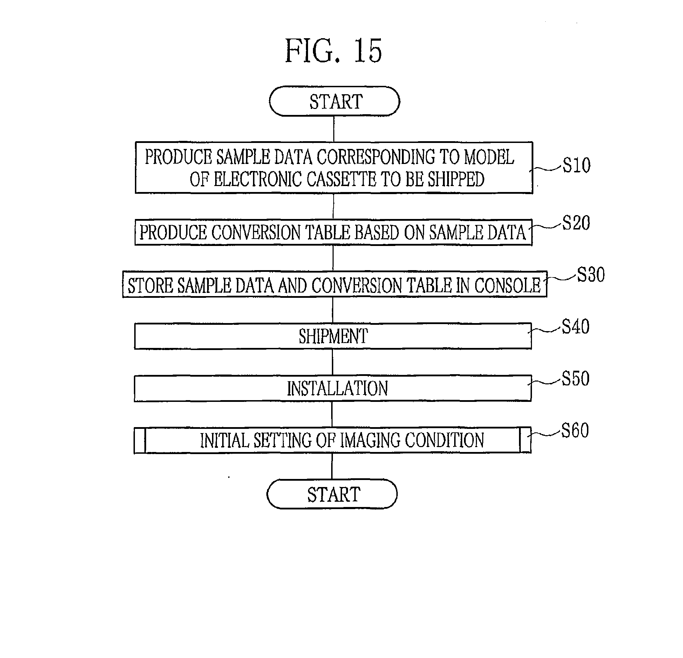

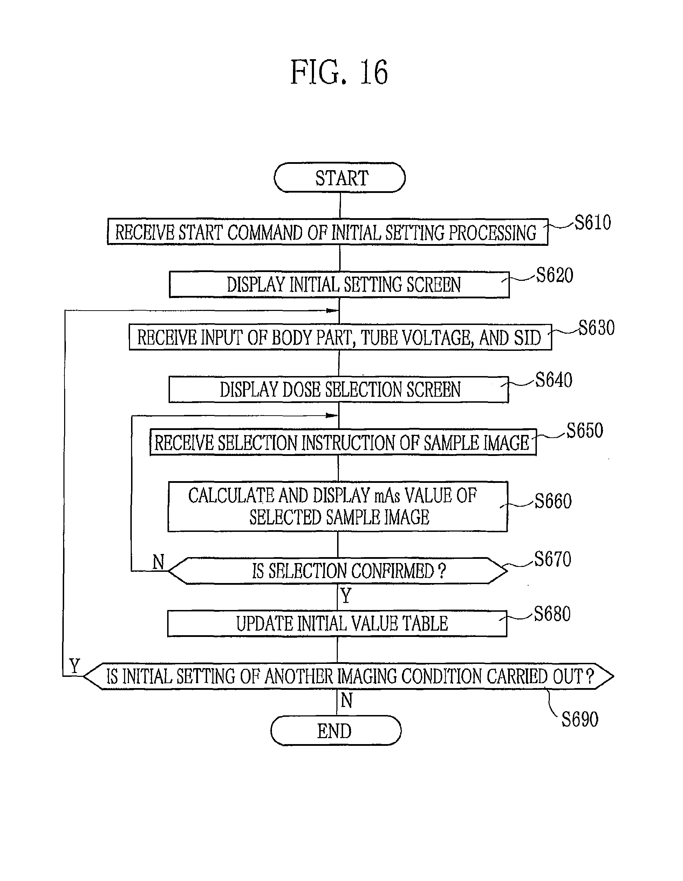

Referring to flowcharts of Figs. 15 and 16, an operation of the X-ray imaging system 10 of the above configuration is described. In Fig. 15, the manufacturer produces the electronic cassette 21 and the sample data 53 for the electronic cassette 21 to be shipped. The sample data 53 is produced on a model by model basis (S10). To produce the sample data 53, X-ray imaging is carried out for two or more times using the electronic cassette 21, with a different dose for each combination of a body part and a tube voltage. Thereby, the sample images are obtained. The sample data 53 is produced based on the sample images.

The S value is obtained from a gray-level histogram of each sample image. The S value is stored in the additional information of the corresponding sample image. The conversion table 54 is produced by associating the S value with the mAs value, being the exposure dose for the imaging (S20). The standard SID used in producing the sample data 53 is stored in the conversion table 54. Thus, the sample data 53 and the conversion table 54 reflect the sensitivity properties of the electronic cassette 21 on a model-by-model basis.

The sample data 53 and the conversion table 54 are stored in the storage device 43 of the console 24 (S30). The console 24 and the electronic cassette 21 are shipped together after the sample data 53 and the conversion table 54 are stored in the console 24 (S40). In the medical institution, the electronic cassette 21 and the console 24 are installed to configure the X-ray imaging system 10 (S50). Then, the software for the console 24 is initiated and the initial setting of the imaging condition is set (S60).

In Fig. 16, when the initial setting button 67 in the examination order screen 61 shown in Fig. 6 is clicked with the pointer 49a, the main controller 41b receives the start command of the initial setting processing (S610). Then, the main controller 41b displays the initial setting screen 71 shown in Fig. 10 on the display 48 through the input and output controller 41a (S620). The model of the electronic cassette 21, the body part, and the tube voltage are inputted into the respective selection boxes 72 to 74 in the initial setting screen 71. The SID used in the medical institution is inputted into the selection box 75. When the OK button 78a is clicked, the main controller 41b receives the model, the body part, the tube voltage, and the SID inputted (S630).

When the dose selection button 77 in the initial setting screen 71 is clicked, the main controller 41b displays the dose selection screen 81 shown in Fig. 11 on the display 48 through the input and output controller 41a (S640). Out of the sample data 53, the main controller 41b selects the image set corresponding to the model, the body part, and the tube voltage inputted (see Fig. 8), and displays the sample images 82a to 82f of the selected image set in a tiled manner in the dose selection screen 81. At the same time, the S values are displayed below the corresponding sample images 82a to 82f, respectively.

The main controller 41b monitors input of a selection instruction through the input and output controller 41a. The selection instruction is inputted when the pointer 49a selects one of the sample images 82a to 82f. When the selection instruction is inputted, the main controller 41b receives the selection instruction (S650). When one of the sample images is selected, the main controller 41b performs the mAs value calculation processing to calculate the mAs value based on the S value of the selected sample image and the inputted SID. The mAs value calculated is displayed in the speech balloon-shaped area 84 close to the selected sample image in the dose selection screen 81 (S660).

In the medical institution, the sample images 82a to 82f are evaluated, and one of the sample images 82a to 82f with the density suitable for the diagnosis is selected. The sample images 82a to 82f are the real images captured by the manufacturer using the electronic cassette 21. Accordingly, the sample images 82a to 82f reflect the sensitivity properties of the electronic cassette 21 more accurately when compared with conventional simulation images that use past images as reference images or basis images. Thus, a more accurate initial value is determined. Additionally, the sample images 82a to 82f displayed in a tiled manner facilitates the comparison thereof.

In the dose selection screen 81, the S value and the mAs value are displayed as information representing the dose used for the selected sample image. When the medical institution employing the IP cassette newly introduces the electronic cassette 21, the initial setting of the imaging condition is carried out immediately after the electronic cassette 21 is introduced. As described above, since the S value is the index also used for the IP cassette, the user in the medical institution is easily accustomed to use the S value as the dose index. Thus, the S value facilitates the determination of the appropriate dose.

The mAs value displayed in the speech balloon-shaped area 84 in the dose selection screen 81 reflects the sensitivity property of the electronic cassette 21 and the SID used in the medical institution where the electronic cassette 21 is installed. Thus, the mAs value, along with the sample image and the S value, is useful in determining the appropriate initial value. For example, when the medical institution sets a desired values to reduce the absorption dose, the initial value is set with comparing the mAs value and the desired value.

The main controller 41b monitors confirming the selected sample image (S670). Namely, the main controller 41b monitors whether the OK button 83a or the apply button 83c is clicked with the pointer 49a. When the selected sample image is confirmed, the main controller 41b stores the mAs value, corresponding to the selected sample image, in the initial value table 52. Thus, the initial value table 52 is updated (S680).

Thereby, the initial value suitable for the medical institution is set in the initial value table 52. To set another initial setting of the imaging condition (for example, the combination of the body part and the tube voltage), the user goes back to the initial setting screen 71, and then selects another imaging condition and repeat the above described processing steps. When the initial settings of the necessary imaging conditions are completed, the user goes back to the examination order screen 61 and exits the initial setting processing (S690).

To perform the X-ray imaging after the initial setting, the tube voltage suitable for the imaging request is inputted through the examination order screen 61. The main controller 41b reads out the mAs value, corresponding to the combination of the body part specified by the imaging request and the inputted tube voltage, from the initial value table 52 and displays the mAs value in the examination order screen 61. When necessary, the operator adjusts the mAs value displayed in the examination order screen 61 in accordance with the physique of the examinee H. The operator sets the adjusted mAs value in the threshold table 14b through the operation panel 14a of the source control device 14. When the radiation switch 15 is pressed, the X-ray source 13 starts application of the X-rays. The X-ray source 13 stops the application of the X-rays when the exposure dose reaches the value set in the threshold table 14b.

Thus, a function to assist in the initial setting of the dose is provided by displaying the sample data 53 that is produced before the shipment the electronic cassette 21. This function allows the user to perform the X-ray imaging with the imaging condition which reduces the exposure dose in accordance with the performance of the electronic cassette 21 when the medical institution newly introduces the electronic cassette 21. Without the function to assist in the initial setting of the dose, the imaging condition suitable for the sensitivity properties of the conventional image detection panel such as the IP cassette tends to be applied to the new electronic cassette 21. The function to assist in the initial setting of the dose solves such tendency and induces the medical institution to reduce the exposure dose. Since the manufacturer produces the sample data 53 before the shipment the electronic cassette 21, there is no need to perform preliminary imaging (test imaging) of the patient (examinee) to determine the imaging condition. Thus, the dose absorption caused by the preliminary imaging is avoided.

In the above example, the S value is converted into the mAs value using the conversion table by way of example. Alternatively, the S value may be converted into the mAs value through calculation using an expression, for example, a function.

The sample data and the conversion table are provided separately by way of example. Alternatively, the conversion table may be integrated into the sample data with the use of the additional information of each sample image, for example. To be more specific, the information to be stored in the conversion table, for example, the standard SID used in capturing the sample image, and the mAs value corresponding to the S value and the standard SID, may be stored in the additional information of each sample image in addition to the S value obtained from the gray-level histogram. When the mAs value is stored in the additional information, the main controller 41b omits the S value/mAs value conversion in the mAs value calculation processing. Instead, the main controller 41b performs only the distance correction to the mAs value read out from the additional information.

The S values are constantly displayed in the dose selection screen 81, namely, the S values are displayed while the sample images are displayed, regardless of whether one of the sample images is selected using the pointer 49a, by way of example. Alternatively, only the S value of the selected sample image may be displayed when one of the sample images is selected using the pointer 49a, in a manner similar to the above mAs value. On the contrary, the mAs values may be displayed constantly in a manner similar to the S values of the above example.

Alternatively, both the S value and the mAs value may be displayed only when one of the sample images is selected using the pointer 49a. In other words, neither the S value nor the mAs value is displayed when the sample image is not selected. Information displayed around the sample image may hinder the evaluation of the image quality of the sample image. To avoid the problem, both the S value and the mAs value may disappear when the selection of the sample image is canceled. Note that an operation button to hide the information representing the dose, for example, the S value and the mAs value, may be provided even if the information representing the dose is displayed constantly.

In the above example, the dose is selected using the sample images displayed in the dose selection screen 81, and the setting in the initial value table 52 is carried out when the selection of the sample image is confirmed, by way of example. Alternatively, the setting in the initial value table 52 may be omitted. For example, when the IP cassette is used as the image detection panel in the X-ray imaging apparatus 12, the setting of the dose, being the imaging condition, may be unnecessary. In this case, the initial value is set only to the threshold table 14b of the X-ray generating device 11. In the above example, the dose (mAs value) is set to the threshold table 14b manually through the operation panel 14a of the source control device 14. In a similar manner, the initial value may be set to the threshold table 14b manually. When the dose (mAs value) corresponding to the selected sample image is displayed in the dose selection screen 81, the operator checks the screen visually and sets the initial value to the threshold table 14b manually.

When the dose is selected by using the sample images and confirmed in the dose selection screen 81, the initial value of the selected dose may be automatically set to the threshold table 14b of the source control device 14 instead of the initial value table 52. In other words, the threshold table 14b is used as the initial value table. In this case, a transmission function is provided to each of the console 24 and the source control device 14 to transmit the information of the dose therebetween. Alternatively, a function to automatically set the information of dose to each of the initial value table 52 and the threshold table 14b may be provided.

The S value is used as the index representing the detected dose by way of example. Instead of the S value, a REX (Reached Exposure index) or an EI (Exposure Index) may be used.

In the above example, the sample data includes two or more sets of sample data corresponding to respective different models of the electronic cassette 21 by way of example. The sample data may include at least a single set of sample data corresponding to the model of the electronic cassette 21 to be introduced to the medical institution. There are advantages in providing the sets of sample data corresponding to the respective different models. As for the manufacturer, it is easy to manage the sample data because this eliminates the need for distinguishing the sample data on the model-by-model basis. This avoids mistakes at the time of the shipment, such as installing the sample data not conforming to the model of the electronic cassette to be shipped. As for the user, the sets of sample data allow the user to control the electronic cassettes of different models with a single console.

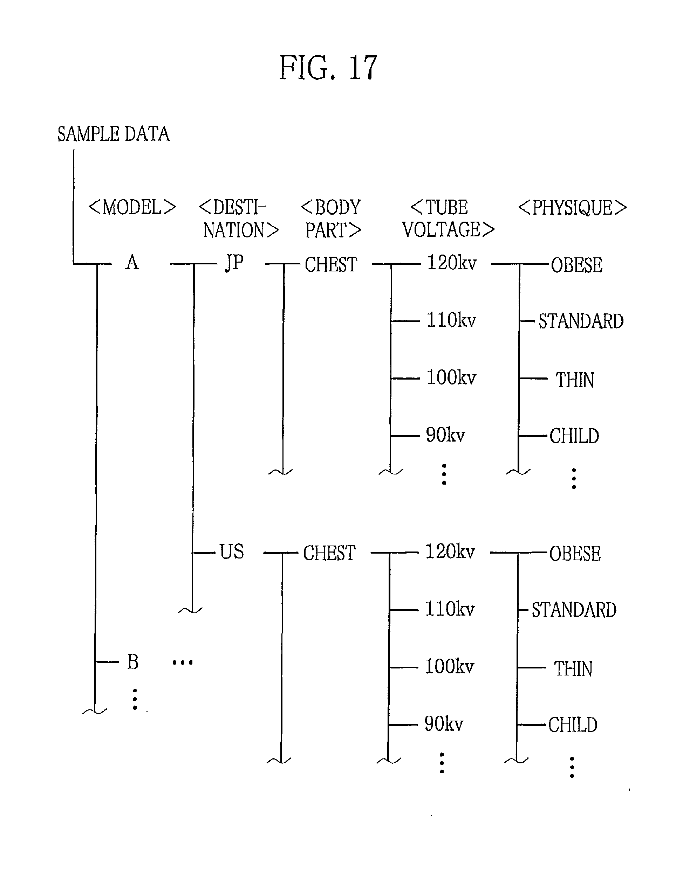

Alternatively, the sample data may be provided on a destination-by-destination basis as shown in Fig. 17. To be more specific, the sample data may be provided on a country-by-country basis, or a region-by-region basis, for example. Each region may include two or more countries, for example, Asia, Europe, or the like. A standard physique of an examinee may vary depending on a destination of shipment (for example, a region) due to different ethnic characteristics. The extent of attenuation of the X-rays varies depending on the physique of the examinee, so that it is necessary to change the standard value of the dose on the destination-by-destination basis of the shipment. It is convenient when the sample data is prepared in advance, taking the differences in physics of the examinees among the destinations into account. In the above example, the initial value is adjusted by multiplying an adjustment rate to the standard value in accordance with the type of the physic, for example, "obese", "standard", "thin", or "child". Alternatively, as shown in Fig. 17, the sample data with the initial values for the different physiques may be prepared in advance. In this case, input boxes for inputting the destination (country, region, or the like) and the physique type are provided in the initial setting screen of the imaging condition. The initial setting (the S value /mAs value conversion, the distance correction, and the like) is carried out in accordance with the inputted conditions.

Alternatively or in addition, the sample data may be prepared on an SID-by-SID basis, a position-by-position basis (for example, lying position or standing position of the examinee), a direction-by-direction basis (for example, A-P or P-A), or a model-by-model basis of the X-ray source 13.

(Second embodiment)

In the second embodiment, an additional function is provided to the X-ray imaging system 10 of the first embodiment. The additional function is to receive the selection instruction of the dose on a doctor-by-doctor basis and display selection statuses of the doctors. The second embodiment is similar to the first embodiment except for the additional function. The additional function is mainly described in the following.

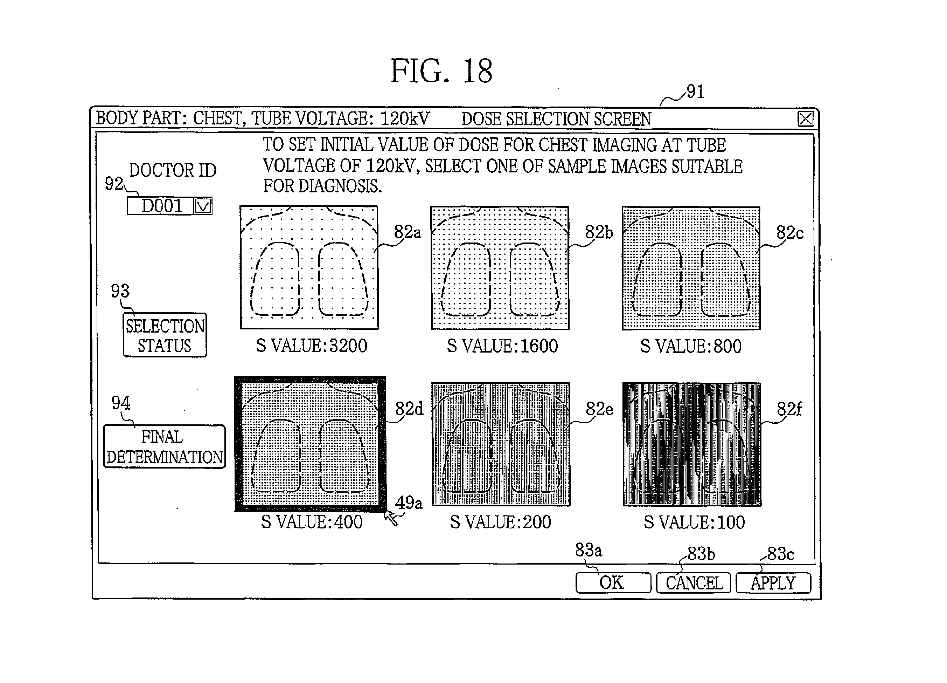

As shown in Fig. 18, a dose selection screen 91 of the second embodiment is provided with a doctor ID input box 92 and a selection status button 93. A doctor ID, being identification information of a doctor, is inputted to the ID input box 92. The main controller 41b receives the selection instruction of the dose, through the sample image selected, in association with the doctor ID inputted to the doctor ID input box 92. The selection instruction is stored in the memory 42 or the storage device 43.

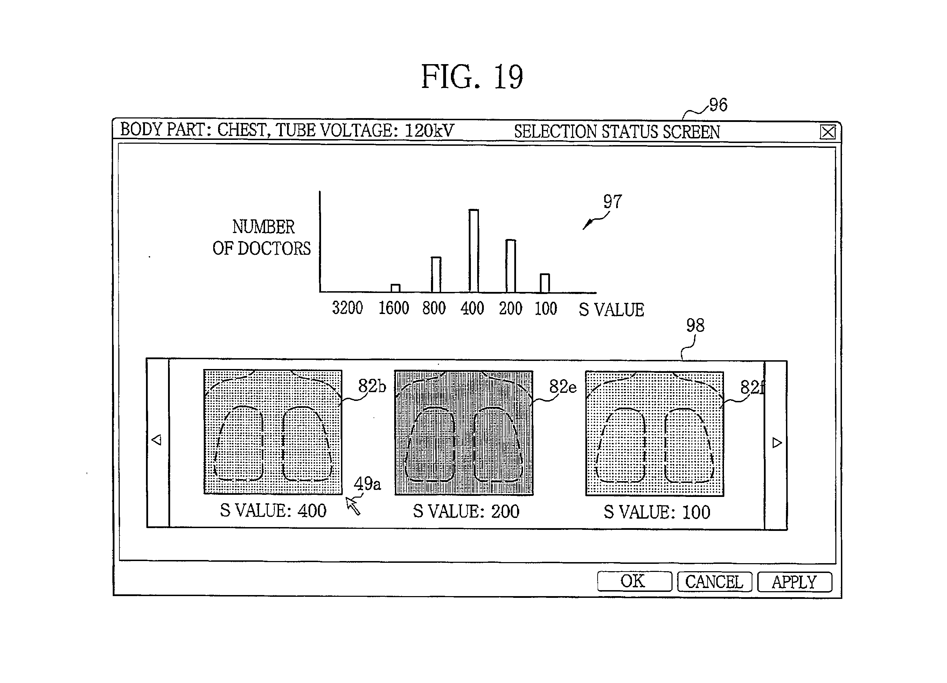

When the selection status button 93 is clicked, the main controller 41b displays a selection status screen 96 on the display 48 as shown in Fig. 19. The selection status screen 96 is provided with a graph 97 and a sample image area 98. The graph 97 shows status of the selection of sample images selected by the doctors. A horizontal axis of the graph 97 represents the dose (the S value). The vertical axis represents the number of the doctors who selected the sample image. Based on the status of the selection, the sample image area 98 shows the sample images in the descending order of the number of the doctors who selected the sample image.

The graph 97 helps to check the dose (density) judged as appropriate by the largest number of the doctors and variations in the doses (densities) judged as appropriate, for example. The medical institution determines the initial value of the dose, taking the status of the selection into account. To be more specific, an average of the doses selected may be used as the initialvalue. Alternatively, the dose (density) selected by the most inexperienced doctor may be used as the initial value because the selected dose may facilitate interpretation of the image. Thus, the status of the selection provides valuable information to determine the initial value of the dose in the medical institution.

In Fig. 18, a final determination button 94 in the dose selection screen 91 is used to finally determine the initial value of the dose. When the final determination button 94 is clicked in a state that one of the sample images is selected, the mAs value corresponding to the selected sample image is stored in the initial value table 52. The final determination button 94 may appear only when an ID of a doctor with authority to finally determine the initial value is inputted.

(Third embodiment)

In a third embodiment, an additional function is provided to the X-ray imaging system of the first or second embodiment. Using the additional function, a result of comparison between a dose used for past image(s) captured with the existing image detection panel (for example, the IP cassette) in the medical institution and a dose used for an image captured with the electronic cassette 21 newly introduced to the medical institution is displayed. The third embodiment is similar to the first or second embodiment except for the additional function. The additional function is mainly described in the following.

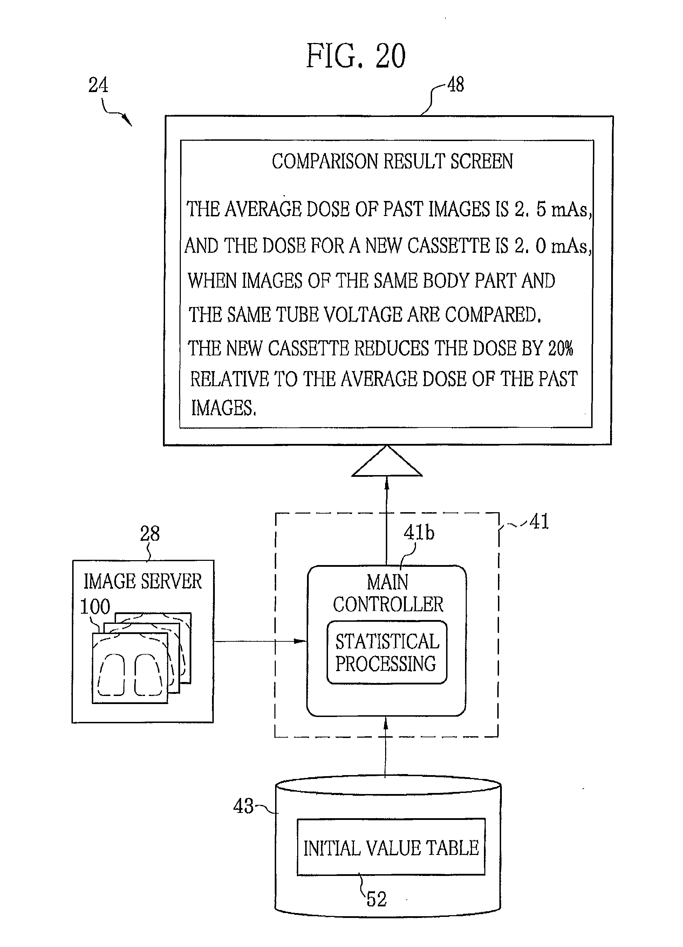

In Fig. 20, when the electronic cassette 21 is newly introduced, the image server 28 contains past images 100 captured using the IP cassette. The main controller 41b obtains two or more past images 100 from the image server 28 to perform statistical processing of the doses of the past images 100 (for example, calculation to obtain an average dose of the past images 100). In more detail, the dose is read out from the imaging condition included in additional information of each past image 100 or the dose is obtained from the gray-level histogram of each past image 100. Then, the initial value of the dose, stored in the initial value table 52 through the above-described initial setting processing after the electronic cassette 21 is introduced to the medical institution, is read out and compared with the average dose of the past images 100, for example. For the comparison, images of the same body part captured at the same tube voltage are used. A rate of reduction in the dose used for the image captured with the electronic cassette 21 relative to the average dose of the past images 100 is calculated.

The main controller 41b displays the result of the comparison on the display 48. The result of the comparison includes the dose from the existing IP cassette and the dose from the new electronic cassette 21, and the rate of reduction of the dose. The result of the comparison shows a specific numerical value indicating the extent of the reduction in absorption dose (or exposure dose) achieved by the use of the new electronic cassette 21, relative to the dose from the existing IP cassette. The comparison result gives the medical institution a strong motive to change the imaging condition, namely, to reduce the dose.

(Fourth Embodiment)

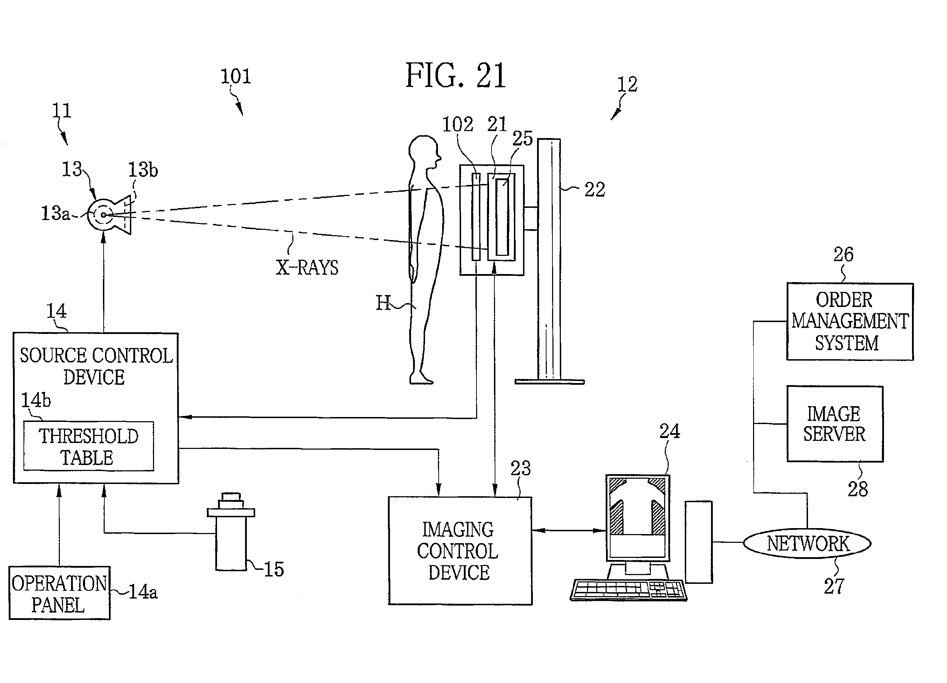

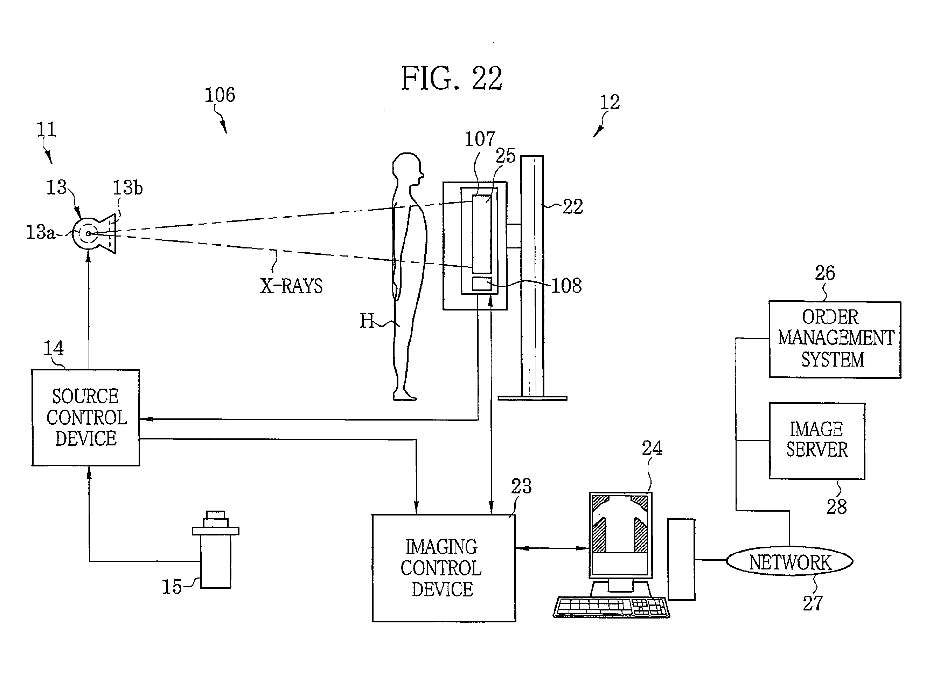

In the X-ray imaging system 10 of the above embodiments, the X-ray source 13 stops the application of the X-rays when the exposure dose reaches a predetermined value, by way of example. Each of X-ray imaging systems 101 and 106 of the fourth embodiment shown in Figs. 21 and 22, on the other hand, is added with an AEC (Auto Exposure Control) function. The AEC function measures the dose, passed through the examinee H and incident on the X-ray imaging apparatus 12, and stops the application of the X-rays from the X-ray source 13 when the incident dose reaches a predetermined threshold value. Other functions are similar to the above embodiments. The AEC function is mainly described in the following.

In Fig. 21, in an X-ray imaging system 101, a phototimer 102 is provided in front of the electronic cassette 21. The phototimer 102 detects the X-rays passed through the examinee H and converts the detected X-rays into an electronic signal. Thus, the phototimer 102 measures the incident dose. Then the phototimer 102 sends a measured value to the source control device 14. The measured value outputted from the phototimer 102 is, for example, an instantaneous value corresponding to the dose per unit time. The source control device 14 integrates the measured values received from the phototimer 102 and monitors an integrated value. When the integrated value reaches the mAs value set in the threshold table 14b, the source control device 14 sends the termination command to the X-ray source 13 to stop the application of the X-rays.

Similar to the first embodiment, the exposure dose (the imaging condition), determined based on the mAs value set in the initial value table 52 of the console 24, is set to the threshold table 14b. In the first embodiment, the mAs value is subjected to the distance correction in the initial setting. When the automatic exposure control is performed as in the X-ray imaging system 101, the dose passed through the examinee H and incident on the phototimer 102 is measured instead of the exposure dose. Accordingly, the distance correction (see Fig. 14) is unnecessary.

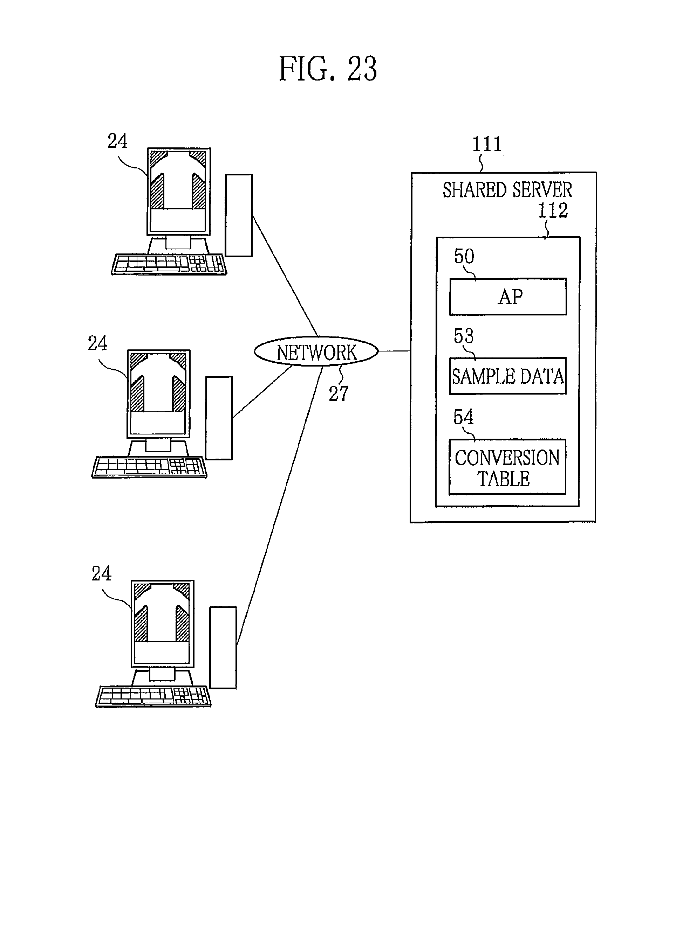

In the X-ray imaging system 101, the phototimer 102 and the source control device 14 constitutes an automatic exposure control device. On the other hand, in the X-ray imaging system 106 shown in Fig. 23, an electronic cassette 107 has a function to measure the dose, similar to that of the phototimer 102. In other words, the electronic cassette 107 itself functions as the automatic exposure control device. In the electronic cassette 107, a part of the pixels 32 functions as detection elements for measuring the dose. The dose (incident dose) incident on the electronic cassette 107 is measured using the detection elements.

The electronic cassette 107 has a threshold table 108 similar to the threshold table 14b. The threshold table 108 is stored in a memory incorporated in the FPD 25, for example. The electronic cassette 107 monitors the incident dose. When the incident dose reaches the value of the dose set in the threshold table 108, the electronic cassette 107 outputs a termination request to the source control device 14 through the imaging control device 23 to stop the application of the X-rays from the X-ray source 13. Upon receiving the termination request, the source control device 14 sends the termination command to the X-ray source 13 to stop the application of the X-rays from the X-ray source 13.

Similar to the X-ray imaging system 101, the X-ray imaging system 106 performs automatic exposure control to measure the incident dose. Accordingly, the distance correction in the initial setting is unnecessary. In the X-ray imaging system 106, the electronic cassette 107 itself measures the incident dose and judges whether the incident dose reaches the threshold value. Accordingly, the S value itself may be used as the threshold value instead of the mAs value. In this case, the conversion of the S value into the mAs value is unnecessary.

In the X-ray imaging system 106, the threshold table 108 is stored in the electronic cassette 107 communicable with the console 24. The X-ray imaging system 106 may be provided with a function to send information of the initial value table 52 to the electronic cassette 107 after the initial setting of the initial value table 52 and to automatically set the information of the initial value table 52 as the initial value in the threshold table 108.

Similar to the X-ray imaging system 106, the X-ray imaging system 101 may be provided with a function to send the initial value of the dose, set in the initial value table 52 of the console 24, to the source control device 14 and to automatically set the initial value of the dose as the initial value in the threshold table 14b. In this case, it is necessary to provide a communication function to each of the console 24 and the source control device 14 to communicate with each other.

(Fifth embodiment)

In the above embodiments, a program for assisting in the initial setting, the sample data 53, and the conversion table 54 are installed in the single console 24 by way of example. Alternatively, a program (AP) 50 for assisting in the initial setting, the sample data 53, and the conversion table 54 may be installed in a data storage unit 112 of a shared server 111, which is described in this embodiment. The shared server 111 is connected to two or more consoles 24 in a communicable manner through the network 27. Each console 24 accesses the shared server 111 using a network application such as a web browser, and downloads the initial setting screen 71 and the dose selection screen 81, for example. Each console 24 sends information of the body part to be imaged and the tube voltage to the shared server 111, and receives the result of the initial setting processing and updates the initial value table 52.

Alternatively, only the program for assisting in the initial setting may be installed on the console 24. The sample data 53 and the conversion table 54 are stored in the shared server 111. In this case, the necessary information is downloaded from the shared server 111.

The above embodiments may be combined as necessary. The present invention may have various configurations as long as they are within the scope of the present invention.

For example, to provide the sample data 53 and the conversion table 54 to the user (medical institution), the manufacturer stored the sample data 53 and the conversion table 54 in the storage device 43 of the console 24. Alternatively, the manufacturer may store the sample data 53 and the conversion table 54 in a removable medium, for example, a DVD medium or a USB memory.

The manufacturer may own the shared server 111 shown in Fig. 24. In this case, the manufacturer accepts the access from the console 24 in each medical institution to provide the information online through a public network such as the Internet. The manufacturer may provide an application service through the network. For example, the shared server 111 owned by the manufacturer performs a part or the whole of the initial setting processing upon a request from the user (client) sent from the console 24. Then, the server 111 sends back a result of the processing to the user.

In the above embodiments, the new image detection panel (the electronic cassette) is introduced to the medical institution using the conventional image detection panel (the IP cassette) by way of example. The present invention is also applicable to a case in which an electronic cassette (or an IP cassette) of a new model is introduced to the medical institution using an electronic cassette (or an IP cassette) of an old model.

In the above embodiments, operation screens such as the initial setting screen and the dose selection screen are described by way of example. Various changes may be made to the operation screens.

In the above embodiments, the electronic cassette, being the X-ray image detection device, the imaging control device, and the console are provided separately by way of example. Alternatively, the electronic cassette, the imaging control device, and the console may be integrated. For example, the electronic cassette may incorporate a function of the imaging control device. The electronic cassette may incorporate a function of the console in addition to the function of the imaging control device.

In the present invention, the console functions as a device for assisting in the initial setting of the imaging condition by way of example. Alternatively, a device other than the console may function as the device for assisting in the initial setting of the imaging condition.

The image detection panel according to the present invention may be a fixed X-ray imaging apparatus in which the FPD is incorporated in an X-ray table.

The present invention is applicable to an imaging system using radiation other than the X-rays, for example, gamma rays.

Various changes and modifications are possible in the present invention and may be understood to be within the present invention.

**Claims**

1. A device (24, 111) for assisting in initial setting of an imaging condition, the device used in a radiation imaging system (10, 101, 106) including a radiation source (13) for applying radiation and an image detection panel (21, 107) for receiving the radiation passed through a subject and detecting a radiation image, the imaging condition including a dose of the radiation applied from the radiation source, the device comprising:

   a data storage means (43, 112) for storing sample data (53), the sample data corresponding to a model of the image detection panel, the sample data including two or more sample images captured with different doses before a shipment of the image detection panel; and
   a display control means (41b) for displaying, on a display, the samples images read out from the data storage means so as to set an initial value of the dose in the initial setting.

2. The device of claim 1, wherein a set of the sample images is prepared for each combination of a body part and a tube voltage, and the sample data includes two or more sets of the sample images, and the tube voltage is supplied to the radiation source and determines radiation quality.

3. The device of claim 2 or 3, wherein the sample data is prepared on a destination-by-destination basis of the shipment.

4. The device of claim 2 or 3, wherein the sample data is prepared for each physique type of the subject.

5. The device of one of claims 1 to 4, wherein the display control means displays a sample image screen (81) on the display, and the sample image screen displays the sample images in a tiled manner.

6. The device of claim 5, wherein the sample image screen displays information representing the dose used for each of the sample images in addition to the sample images.

7. The device of one of claims 1 to 6, wherein the sample data includes an S value calculated from a gray-level histogram of each of the sample images.

8. The device of claim 7, further comprising a conversion means (54) for converting the S value into an mAs value, being a product of a tube current supplied to the radiation source and exposure time.

9. The device of claim 8, further comprising:

   an SID receiving means (41b) for receiving input of an SID, being a distance between the image detection panel and the radiation source; and
   a correction means (41b) for correcting the mAs value based on the inputted SID and a standard SID used for imaging the sample image.

10. The device of one of claims 1 to 9, further comprising a selection instruction receiving means (41b) for receiving input of a selection instruction that selects one of the sample images.

11. The device of claim 10, wherein the display control means displays, on the display, at least information representing the dose of the sample image selected through the selection instruction receiving means.

12. A method for assisting in initial setting of an imaging condition, the method being used in a radiation imaging system (10, 101, 106) including a radiation source (13) for applying radiation and an image detection panel (21, 107) for receiving the radiation passed through a subject and detecting a radiation image, the imaging condition including a dose of the radiation applied from the radiation source, the method comprising the steps of:

   reading out two or more sample images from a data storage means (43, 112), the data storage means storing sample data (53), the sample data corresponding to a model of the image detection panel, the sample data including the sample images captured with different doses before a shipment of the image detection panel; and
   displaying the read-out sample images on a display (48) to set an initial value of the dose in the initial setting.

13. A program (50) for assisting in initial setting of an imaging condition, the program being used in a radiation imaging system (10, 101, 106) including a radiation source (13) for applying radiation and an image detection panel (21, 107) for receiving the radiation passed through a subject and detecting a radiation image, the imaging condition including a dose of the radiation applied from the radiation source, the program comprising the steps of:

reading out two or more sample images from a data storage means (43, 112), the data storage means storing sample data (53), the sample data corresponding to a model of the image detection panel, the sample data including the sample images captured with different doses before a shipment of the image detection panel; and displaying the read-out sample images on a display (48) to set an initial value of the dose in the initial setting.

14. A radiation imaging apparatus (12) including an image detection panel (21, 107), the image detection panel receiving radiation applied from a radiation source (13) and passed through a subject and detecting a radiation image, the radiation imaging apparatus comprising:

a console (24) for setting an imaging condition including a dose of the radiation applied from the radiation source, the console including a data storage means (43) and a display control means (41b), the data storage means storing sample data (53), the sample data corresponding to a model of the image detection panel, the sample data including two or more sample images captured with different doses before a shipment of the image detection panel, the display control means displays, on a display (48), the sample images read out from the data storage means so as to set an initial value of the dose.

15. A radiation imaging system (10, 101, 106) including a radiation source (13) for applying radiation and an image detection panel (21, 107) for receiving the radiation passed through a subject and detecting a radiation image, the radiation imaging system comprising:

a console (24) for setting an imaging condition including a dose of the radiation applied from the radiation source, the console including a data storage means (43) and a display control means (41b), the data storage means storing sample data (53), the sample data corresponding to a model of the image detection panel, the sample data including two or more sample images captured with different doses before a shipment of the image detection panel, the display control means displays, on a display (48), the sample images read out from the data storage means so as to set an initial value of the dose.

FIG. 1

# FIG. 2

| X-RAY SOURCE | STOP | IRRADIATE | STOP |
|---|---|---|---|

START COMMAND | TERMINATION COMMAND

RADIATION SWITCH ON OFF

MONITOR DOSE

SOURCE CONTROL DEVICE

SYNCHRONIZATION START SIGNAL | SYNCHRONIZATION STOP SIGNAL

| X-RAY IMAGING APPARATUS (ELECTRONIC CASSETTE) | STANDBY | ACCUMULATE SIGNALS | READ |
|---|---|---|---|

EP 2 574 280 A2

# FIG. 3

COLUMN    ROW     25

33

31

32

# FIG. 4

24

DISPLAY ~48

INPUT DEVICE ~49

41

CPU

47

MEMORY ~42

43

STORAGE DEVICE

AP ~50

COMMUNICATION I/F ~44

27

NETWORK

# FIG. 5

INPUT AND OUTPUT CONTROLLER — 41a

OPERATION SCREEN

· BODY PART
· TUBE VOLTAGE
· SID
· DOSE

41b

MAIN CONTROLLER

IMAGING CONDITION SETTING PROCESSING

INITIAL SETTING PROCESSING

COMMUNICATION I/F — 44

48

49

INPUT DEVICE

43

53 — SAMPLE DATA

54 — CONVERSION TABLE

52 — INITIAL VALUE TABLE

51 — ORDER STORAGE AREA

IMAGE SET (CHEST, 120kV)
IMAGE SET (CHEST, 110kV)
IMAGE SET (CHEST, 100kV)
IMAGE SET (CHEST, 90kV)
⋮

CONVERSION TABLE (S VALUE:1600)
CONVERSION TABLE (S VALUE:800)
CONVERSION TABLE (S VALUE:600)
CONVERSION TABLE (S VALE:400)
⋮

IMAGING REQUEST
— IMAGING CONDITION
— X-RAY IMAGE

EP 2 574 280 A2

# FIG. 6

EXAMINATION ORDER ID:1021  PATIENT ID: 001  NAME : TARO FUJI  GENDER: MALE  AGE: 50

IMAGING ORDER 1
CHEST, STANDING, FRONT (P→A)

IMAGING ORDER 2
STOMACH, STANDING, FRONT (P→A)

kV
120

mAs
2. 46

STANDARD ☑

INITIAL SETTING

OK   CANCEL   EXIT

EP 2 574 280 A2

# FIG. 7

52

| BODY PART | TUBE VOLTAGE (kV) | mAs VALUE |
|---|---|---|
| CHEST | 120 | 2. 46 |
| | 110 | 3. 08 |
| | 100 | 3. 69 |
| | 90 | 4. 31 |
| | 80 | 4. 92 |
| STOMACH | 120 | 1. 77 |
| | 110 | 2. 46 |

# FIG. 8

SAMPLE DATA

<MODEL> <BODY PART> <TUBE VOLTAGE>

A — CHEST — 120kv

IMAGE SET (CHEST, 120kV)

~ SAMPLE IMAGE

| ADDITIONAL INFORMATION (INCLUDING S VALUE) |
| IMAGE DATA |

110kv

IMAGE SET (CHEST, 110kV)

100kv

90kv

IMAGE SET (CHEST, 100kV)

HEAD — 100kv

IMAGE SET (CHEST, 90kV)

80kv

B

EP 2 574 280 A2

# FIG. 9

# FIG. 10

**INITIAL SETTING SCREEN FOR IMAGING CONDITIONS** ☒

71

| MODEL | BODY PART | TUBE VOLTAGE | SID |
|---|---|---|---|
| 72 | 73 | 74 | 75 |

MODEL: A ▽

BODY PART: HEAD, CHEST, STOMACH, LUMBAR SPINE ⋮ ▽  49a

TUBE VOLTAGE: 120 ▽ kV

SID: 200 . cm

77  SELECT DOSE

mAs VALUE: 2.46  79

78a 78b 78c

OK  CANCEL  APPLY

# FIG. 11

**81**

BODY PART: CHEST,  TUBE VOLTAGE: 120kV        DOSE SELECTION SCREEN        ☒

TO SET INITIAL VALUE OF DOSE FOR CHEST IMAGING AT TUBE VOLTAGE OF 120kV,
SELECT ONE OF SAMPLE IMAGES SUITABLE FOR DIAGNOSIS

82a

82b

82c

84

mAs VALUE:2. 46
WHEN SID:200cm

S VALUE: 1600        S VALUE: 800        S VALUE: 600

82d

82e

82f

49a

S VALUE: 400        S VALUE: 200        S VALUE: 100

83a        83b        83c

OK        CANCEL        APPLY

EP 2 574 280 A2

# FIG. 12

$$S = 4 \times 10^{4-Qk}$$

$$S1 > S2$$

# FIG. 13

54

| STANDARD SID:180(cm) | | | |
|---|---|---|---|
| S VALUE | BODY PART | TUBE VOLTAGE (kV) | mAs VALUE (PRODUCT OF TUBE CURRENT AND TIME) |
| 400 | CHEST | 120 | 2 |
| | | 110 | 2. 5 |
| | | 100 | 3 |
| | | 90 | 3. 5 |
| | | 80 | 4 |
| | LUMBAR SPINE | 80 | 20 |
| | FOUR LEG BONES | 50 | 10 |
| 600 | CHEST | 120 | 1. 44 |

# FIG. 14

| STD SID:180(cm) — 54 | | | |
|---|---|---|---|
| S VALUE | BODY PART | TUBE VOLTAGE (kV) | mAs VALUE |
| 400 | CHEST | 120 | 2 |
| ⋮ | ⋮ | ⋮ | ⋮ |

DISTANCE CORRECTION PROCESSING

$$\text{mAs VALUE (I)} = \text{mAs VALUE (I0)} \times 1 / (\text{SID0} / \text{SID})^{2}$$

| — 52 | | | | |
|---|---|---|---|---|
| BODY PART | TUBE VOLTAGE (kV) | S VALUE | SID (cm) | mAs VALUE |
| CHEST | 120 | 400 | 200 | 2. 46 |
| ⋮ | ⋮ | ⋮ | ⋮ | ⋮ |

14

14b

THRESHOLD TABLE

EP 2 574 280 A2

# FIG. 15

```
                    ┌─────────────┐
                    │    START    │
                    └──────┬──────┘
                           │
┌──────────────────────────────────────────────────────┐
│  PRODUCE SAMPLE DATA CORRESPONDING TO MODEL            │  ~S10
│       OF ELECTRONIC CASSETTE TO BE SHIPPED             │
└──────────────────────────┬───────────────────────────┘
                           │
┌──────────────────────────────────────────────────────┐
│  PRODUCE CONVERSION TABLE BASED ON SAMPLE DATA         │  ~S20
└──────────────────────────┬───────────────────────────┘
                           │
┌──────────────────────────────────────────────────────┐
│  STORE SAMPLE DATA AND CONVERSION TABLE IN CONSOLE     │  ~S30
└──────────────────────────┬───────────────────────────┘
                           │
┌──────────────────────────────────────────────────────┐
│                     SHIPMENT                           │  ~S40
└──────────────────────────┬───────────────────────────┘
                           │
┌──────────────────────────────────────────────────────┐
│                   INSTALLATION                         │  ~S50
└──────────────────────────┬───────────────────────────┘
                           │
┌──────────────────────────────────────────────────────┐
│          INITIAL SETTING OF IMAGING CONDITION          │  ~S60
└──────────────────────────┬───────────────────────────┘
                           │
                    ┌──────┴──────┐
                    │    START    │
                    └─────────────┘
```

# FIG. 16

START

RECEIVE START COMMAND OF INITIAL SETTING PROCESSING ～S610

DISPLAY INITIAL SETTING SCREEN ～S620

RECEIVE INPUT OF BODY PART, TUBE VOLTAGE, AND SID ～S630

DISPLAY DOSE SELECTION SCREEN ～S640

RECEIVE SELECTION INSTRUCTION OF SAMPLE IMAGE ～S650

CALCULATE AND DISPLAY mAs VALUE OF SELECTED SAMPLE IMAGE ～S660

N    IS SELECTION CONFIRMED ? ～S670

Y

UPDATE INITIAL VALUE TABLE ～S680

Y    IS INITIAL SETTING OF ANOTHER IMAGING CONDITION CARRIED OUT ?

N                                    ～S690

END

# FIG. 17

SAMPLE DATA

| \<MODEL\> | \<DESTI-NATION\> | \<BODY PART\> | \<TUBE VOLTAGE\> | \<PHYSIQUE\> |
|---|---|---|---|---|
| A | JP | CHEST | 120kv | OBESE |
| | | | 110kv | STANDARD |
| | | | 100kv | THIN |
| | | | 90kv | CHILD |
| | | | ⋮ | ⋮ |
| | US | CHEST | 120kv | OBESE |
| | | | 110kv | STANDARD |
| | | | 100kv | THIN |
| B  ⋯ | | | 90kv | CHILD |
| ⋮ | | | ⋮ | ⋮ |

# FIG. 18

BODY PART: CHEST, TUBE VOLTAGE: 120kV     DOSE SELECTION SCREEN     ⊠

TO SET INITIAL VALUE OF DOSE FOR CHEST IMAGING AT TUBE VOLTAGE OF 120kV, SELECT ONE OF SAMPLE IMAGES SUITABLE FOR DIAGNOSIS.

DOCTOR ID
D001

SELECTION STATUS

FINAL DETERMINATION

S VALUE:3200   S VALUE:1600   S VALUE:800

S VALUE:400   S VALUE:200   S VALUE:100

OK   CANCEL   APPLY

# FIG. 19

BODY PART: CHEST, TUBE VOLTAGE: 120kV    SELECTION STATUS SCREEN    ⊠

96

NUMBER OF DOCTORS

97

3200  1600  800  400  200  100    S VALUE

98

82b

82e

82f

49a

S VALUE: 400    S VALUE: 200    S VALUE: 100

OK    CANCEL    APPLY

EP 2 574 280 A2

# FIG. 20

24

48

COMPARISON RESULT SCREEN

THE AVERAGE DOSE OF PAST IMAGES IS 2. 5 mAs,
AND THE DOSE FOR A NEW CASSETTE IS 2. 0 mAs,
WHEN IMAGES OF THE SAME BODY PART AND
THE SAME TUBE VOLTAGE ARE COMPARED.
THE NEW CASSETTE REDUCES THE DOSE BY 20%
RELATIVE TO THE AVERAGE DOSE OF THE PAST
IMAGES.

28

IMAGE SERVER
100

41

41b

MAIN
CONTROLLER

STATISTICAL
PROCESSING

43

INITIAL VALUE TABLE

52

# FIG. 21

EP 2 574 280 A2

## FIG. 22

# FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7949098 B